# EUROPEAN PATENT SPECIFICATION

(11) **EP 2 996 578 B1**
(45) Date of publication and mention of the grant of the patent: **02.08.2017**
(21) Application number: 14715729.1
(22) Date of filing: 03.03.2014
(51) Int. Cl.: A61B 17/064, A61B 17/068, A61F 2/00, A61B 17/10, A61B 50/00, A61B 17/00, A61B 17/062, A61B 50/20

(54) **APPLICATOR SYSTEMS FOR SURGICAL FASTENERS**
APPLIKATORSYSTEME FÜR CHIRURGISCHE BEFESTIGUNGSELEMENTE
SYSTÈMES D'APPLICATEUR POUR ATTACHES CHIRURGICALES

(30) Priority: 14.03.2013 US 201361784497 P; 11.02.2014 US 201414177894
(43) Date of publication of application: 23.03.2016
(73) Proprietor: Ethicon, Inc, Somerville, NJ 08876 (US)
(72) Inventor: MIKSZA, Anthony, Nazareth, Pennsylvania 18064 (US); NERING, Robert, Sergeantsville, N.J. 08557 (US); KENYON, Mark D., Ringoes, New Jersey 08551 (US); VAKHARIA, Omar J., Cincinnati, Ohio 45230 (US); JAMIOLKOWSKI, Dennis D., Long Valley, New Jersey 07853 (US)
(74) Representative: Carpmaels & Ransford LLP
(86) International application number: PCT/US2014/019796
(87) International publication number: WO 2014/158745

(56) References cited:
- WO-A1-2005/099592
- WO-A2-2004/032763
- WO-A2-2008/005465
- US-A1- 2005 107 807
- US-A1- 2005 149 064
- US-A1- 2011 079 627

## Description

### BACKGROUND OF THE INVENTION

### Field of the Invention

The present invention generally relates to securing implants to tissue, and more specifically relates to systems and devices that utilize surgical fasteners for securing implants to tissue.

### Description of the Related Art

Hernia is a condition where a small loop of bowel or intestine protrudes through a weak place or defect within the abdominal muscle wall or groin of a patient. This condition commonly occurs in humans, particularly males. Hernias of this type may result from a congenital defect whereby the patient is born with this problem, or may be caused by straining or lifting heavy objects. Heavy lifting may be known to create a large amount of stress upon the abdominal wall and can cause a rupture or tearing at a weak point of the abdominal muscle to create the defect or opening. In any case, the patient may be left with an unsightly bulge of intestinal tissue protruding through the defect, which may result in pain, reduced lifting abilities, and in some cases, impaction of the bowel, or possibly other complications if the flow of blood is cut off to the protruding tissue.

A common solution to the above-described problem may be surgery. During a surgical procedure, the defect is accessed and carefully examined, either through an open incision or endoscopically through an access port such as a trocar. In either case, careful examination is required due to the network of vessels and nerves which exist in the area of a typical defect, which requires a surgeon to conduct a hernia repair with great skill and caution. Within this area can be found vascular structures such as gastric vessels, the external iliac vessels, and the inferior epigastric vessels, as well as reproductive vessels such as the vas deferens extending through the inguinal floor.

Once the surgeon is familiar with the anatomy of a patient, the surgeon carefully places the viscera back into the patient's abdomen through the defect. Repairing the defect can involve closure of the defect with sutures or fasteners but generally involves placing a surgical prosthetic such as a mesh patch over the open defect, and attaching the mesh patch to the abdominal wall or inguinal floor using a conventional suture or surgical fasteners. The mesh patch acts as a barrier and prevents expulsion of bowel through the defect. Suturing of the mesh patch to the inguinal floor can be well suited to open procedures but can be much more difficult and time consuming with endoscopic procedures. With the adoption of endoscopic surgery, endoscopic surgical instruments that apply surgical fasteners can be used. However, the tissue of the inguinal floor may offer special challenges to the surgeon when a needle or fastener is used to penetrate structures such as Cooper's ligament.

At present, there are a variety of surgical instruments and fasteners available for the surgeon to use in an endoscopic or open procedure to attach the mesh patch to the inguinal floor. One of the earliest types of endoscopic surgical instruments used is a surgical stapler. A plurality or stack of these unformed staples may be generally contained within a stapling cartridge in a serial fashion, and may be sequentially advanced or fed within the instrument by a spring mechanism. A secondary valving or feeding mechanism may be employed to separate the distal most staple from the stack, to hold the remainder of the spring loaded stack, and may be used to feed the distal most staples into the staple forming mechanism. Feeding mechanisms of this type are found in U.S. Patent No. 5,470,010 to Rothfuss et al., and in U.S. Patent No. 5,582,616, also to Rothfuss et al.

US 2005/149064 discloses a device including an applicator assembly configured to deploy a fastener in a first direction, a tissue manipulation assembly configured to move from a relaxed position to a grasping position in a second direction transverse to the first direction, and a translating trigger assembly coupled to the applicator assembly and the tissue manipulation assembly. Another hernia mesh attachment instrument uses a helical wire fastener that resembles a small section of spring. Multiple helical wire fasteners may be stored serially within the 5 mm shaft, and may be corkscrewed or rotated into tissue. A load spring may be used to bias or feed the plurality of helical fasteners distally within the shaft. A protrusion extends into the shaft to possibly prevent the ejection of the stack of fasteners by the load spring and may permit passage of a rotating fastener. Instruments and fasteners of these types are found in U.S. Patent No. 5,582,616 to Bolduc et al., U.S. Patent No. 5,810,882 to Bolduc et al., and in U.S. Patent No. 5,830,221 to Stein et al.

Whereas the above surgical instruments may be used for hernia fastening applications, they use a spring mechanism to feed a plurality of fasteners through the surgical instrument. Spring mechanisms typically use a long soft coil spring to push a stack of fasteners through a guide or track within the shaft of the surgical instrument. These types of feeding mechanisms may be generally simple and reliable, but may require an additional secondary valving mechanism or protrusion to separate and feed one fastener from the stack.

Other surgical fasteners may be used for hernia mesh attachment but utilize either a reloadable single shot instrument or a rotary magazine that holds a small number of fasteners. These types of surgical fastening instruments can be found in U.S. Patent No. 5,203,864 and U.S. Patent No. 5,290,297, both to Edward Phillip. These instruments have not gained acceptance by the surgical community, possibly due to their single shot capabilities and the large size of the rotary magazine, which can restrict such an instrument to an open procedure.

Whereas all the above surgical instruments may be used for hernia fastening applications, they either use a spring mechanism to feed the plurality of fasteners through the surgical instrument, or a rotary magazine in lieu of a feeding mechanism. Other types of surgical fasteners may be available, such as surgical clips, and they can utilize feeding mechanisms that do not require the use of a spring to feed the clips distally. A reciprocating feeding mechanism is described in U.S. Patent Nos. 5,601,573; 5,833,700; and 5,921,997 to Fogelberg et al. The Fogelberg et al. references teach a clip applier with a feeding mechanism that utilizes a reciprocating feed bar to feed a serial stack of clips. A feeder shoe may operably engage with and move with the distally moving feed bar and may slidingly engage with the proximally moving feed bar. Thus, the feeder shoe may index or push the stack of clips distally with the distally moving feed bar and remains stationary relative to the proximally moving feed bar. A valving mechanism may be also required to separate the distal-most clip from the stack and to hold the stack stationary as the distal most clip may be applied onto a vessel. Whereas the Fogelberg et al. references teach a reciprocating feeding mechanism with a single reciprocating member, they do not teach the use of the clip applier in the attachment of hernia mesh, nor do they teach the individual driving or feeding of each clip by a moving member.

Another fastener feeding mechanism that uses reciprocation is that disclosed in U.S. Patent No. 4,325,376 to Klieman et al. A clip applier that stores a plurality of clips in a serial fashion within a clip magazine is disclosed. The clips are in a stack wherein the proximal most clip may be pushed or fed distally by a pawl that may be ratcheted or indexed distally by a reciprocating member or ratchet blade with each actuation of the instrument. As the pawl indexes distally, it can push the stack of clips distally. A secondary valving mechanism may be also described. Thus, the feeding mechanism of Klieman et al. teaches the use a single reciprocating member and pawl to push or feed the stack of clips distally, and may require a secondary valving mechanism to feed the distal most clip.

U.S. Patent No. 3,740,994 to DeCarlo Jr. describes a novel reciprocating feeding mechanism that may index a plurality of staples or clips, and may ready them for discharge by reciprocating one of a pair of opposing leaf spring assemblies. The staples reside serially within a guide rail with a fixed leaf spring assembly extending into the plane of the guide rail. A reciprocating leaf spring assembly may opposedly extend inwardly towards the fixed leaf spring assembly. As the reciprocating leaf spring assembly moves distally, each of individual leaf springs of the assembly may engage a staple and move it distally. The distally moving staples deflect the local individual leaf springs of the fixed leaf spring assembly, and the deflected leaf springs may return to the un-deflected position after passage of the staple. As the moving leaf spring assembly moves proximally, the leaf springs of the fixed leaf spring assembly hold the staples stationary and prevent proximal movement thereof. A secondary guide rail and valving mechanism may be provided to separate a single staple from the stack for forming and can hold the stack of staples stationary as the single clip is formed.

Additionally, similar feeding mechanisms are disclosed in U.S. Patent No. 4,478,220 to DiGiovanni et al. and U.S. Patent No. 4,471,780 to Menges et al. Both of these related patents teach a reciprocating feeding mechanism that uses one fixed member and one reciprocating member to feed or index a plurality of clips distally. Angled flexible fingers may be hingedly attached to the reciprocating member and operatively engage the clips when moving distally, and slidingly engage with the clips when moving proximally. The angled flexible fingers within the fixed member deflect out of the way when the clips move distally and spring up to stop proximal movement of the clip after the clip has passed. A secondary valving mechanism is also disclosed.

Commonly assigned U.S. Patent Application Publication No. 2002/0068947, teaches a device for delivering a plurality of individual surgical fasteners. In one embodiment, the delivery device includes a drive mechanism having distal and proximal ends. The drive mechanism has a moving member and a fixed opposing member, whereby the moving member is moveable proximally and distally with respect to the delivery device. The moving member has a sharpened distal end for piercing tissue. The device includes at least one surgical fastener located between the first and the second members. Each of the at least one surgical fasteners has a proximal end and a distal end. The device also has an actuator having at least two sequential positions. A first position for moving the moving member distally and piercing tissue, and a second position for moving the moving member proximally, thereby deploying the distal end of the fastener.

Tacks for fixing meshes used laparoscopically have generally been made of metal, such as stainless steel, nitinol, or titanium. The metal tacks were necessary to provide for sufficient holding strength, penetration of various prosthetic meshes, and for ease of manufacture. Until recently, there were no absorbable tacks available on the market, and surgeons could only use absorbable sutures in order to provide a fixation means that did not permanently stay in the body. However, using sutures is exceedingly difficult for laparoscopic procedure, and so they are generally not used unless the repair is done in an open fashion. With surgical trends leading to more minimally invasive techniques with minimum foreign body accumulation, an absorbable tack with minimum profile that can be applied laparoscopically is needed.

In spite of the above advances, there remains a need for applicator systems for fixing implants using surgical fasteners whereby the surgical fasteners 1) have a minimum profile, 2) may be applied laparoscopically, and 3) are absorbable. There also remains a need for applicator systems for surgical fasteners that are economical, that use cartridges pre-loaded with surgical fasteners having 1) different sizes, 2) different material compositions, 3) different quantities, and 4) that provide surgical fasteners having curved legs to enable shallower implant fixation. Moreover, there remains a need for applicator systems for surgical fasteners that 1) provide the user with a one-to-one tactile feel when inserting a surgical fastener into tissue, 2) provide the user with manual control over the amount of insertion force, 3) enable the user to push the surgical fastener further into tissue, if desired, and 4) provide the user with a broad range of mechanical fixation capabilities that are similar to those available when using sutures.

### SUMMARY OF THE INVENTION

The invention is defined in the appended claims. In one embodiment, an applicator system for inserting surgical fasteners preferably includes a manually controlled insertion tool having a distal end with an insertion fork that is adapted to slide over the legs of a surgical fastener for loading the surgical fastener onto the insertion fork. The loaded insertion fork is then utilized for inserting the surgical fastener into tissue for securing an implant, such as a mesh implant, to the tissue.

In one embodiment, the applicator system preferably includes one or more cartridges, each of which have a plurality of surgical fasteners pre-loaded therein. In one embodiment, the cartridges have a plurality of elongated slots and a single surgical fastener accessible through each elongated slot. The surgical fasteners are preferably removable from the cartridge for being inserted into tissue for securing an implant, such as a surgical mesh, to the tissue. In one embodiment, in order to remove a surgical fastener from the cartridge, an insertion fork is inserted into one of the elongated slots for loading one of the surgical fasteners onto the insertion fork. After loading the surgical fastener onto the insertion fork, the insertion fork may be removed from the elongated slot and transferred to a surgical site for being manually inserted into tissue by the insertion fork.

In one embodiment, the applicator system disclosed herein incorporates one or more features disclosed in commonly assigned U.S. Patent Appln. Publication Nos. US 2010/0292715, US 2010/0292712, US 2010/0292710, US 2010/0292713, and US 2011/079627, U.S. Patent Application Ser. No. 13/470,022, filed May 11, 2012, entitled "APPLICATOR INSTRUMENTS FOR DISPENSING SURGICAL FASTENERS DURING OPEN REPAIR PROCEDURE", U.S. Patent Appln. Ser. No. 13/470,065, filed on May 11, 2012, entitled "APPLICATOR INSTRUMENTS HAVING DISTAL END CAPS FOR FACILITATING THE ACCURATE PLACEMENT OF SURGICAL FASTENERS DURING OPEN REPAIR PROCEDURES", and U.S. Patent Appln. Ser. No. 13/791,950, filed March 9, 2013, entitled "SURGICAL FASTENERS HAVING ARTICULATING JOINTS AND DEFLECTABLE TIPS,".

In contrast to mesh fixation systems having gun-like insertion tools that automatically dispense a fastener each time a trigger is pulled, the present application discloses a versatile manual system that enables an insertion fork to be manually engaged for handling only one surgical fastener at a time.

The manual system disclosed herein provides a number of benefits. In one embodiment, the insertion tool provides the user with a one-to-one tactile feel when manually inserting a surgical fastener into tissue.

In one embodiment, because the applicator system is manual and not automatic, the insertion tool enables a user to manually control and adjust the amount of insertion force used when inserting a surgical fastener into tissue.

In one embodiment, the manual system enables a user to push a surgical fastener further into tissue, if desired.

The present application also preferably provides more versatility over the types of surgical fasteners that may be inserted into the tissue. For example, the size, configuration and type of surgical fastener can be easily changed during a surgical procedure. This is an advantage over automatic applicator guns that only dispense one type of surgical fastener.

In one embodiment, the insertion tool may include a stored energy element that provides a level of insertion force when using the insertion fork to insert a surgical fastener in tissue. In one embodiment, the insertion tool uses only manual energy provided by the user. In one embodiment, the insertion tool combines the manual energy provided by the user with the energy from the stored energy element to provide insertion force for inserting a surgical fastener in tissue.

In one embodiment, the insertion tool preferably includes a safety release element that prevents the use of excessive insertion force when inserting a surgical fastener into tissue. In one embodiment, the safety release element may be coupled with the elongated shaft 46 or the insertion fork 52 to prevent the use of excessive force during insertion of a surgical fastener. In one embodiment, the shaft 46 or the insertion fork 50 will collapse upon reaching a pre-set or pre-determined level of force. In one embodiment, the safety release element may include a spring that is tripped when a pre-set level of force is reached to prevent over insertion of a surgical fastener, or the use of excessive insertion force.

In one embodiment, a single cartridge may be loaded with surgical fasteners having different sizes and/or properties. In one embodiment, multiple cartridges may be used whereby each cartridge is loaded with surgical fastener having a particular size and/or property.

In one embodiment, one or more cartridges may be loaded with surgical fasteners made of different materials, such as a first cartridge loaded with surgical fasteners that are absorbable, a second cartridge loaded with surgical fasteners that are non-absorbable, a third cartridge loaded with surgical fasteners having straight legs, and a fourth cartridge loaded with surgical fasteners having curved legs.

In one embodiment, surgical fasteners having different sizes, shapes, configurations, flexibility, materials, and other properties may be contained within a single cartridge. In one embodiment, a plurality of cartridges may be provided, whereby each cartridge contains a plurality of surgical fasteners having the same properties, e.g., size, shape, configuration, flexibility, materials, etc. In one embodiment, the cartridges and/or the surgical fasteners may be color coded or have indicia provided thereon to indicate the properties of the surgical fasteners contained within the cartridges.

In one embodiment, the cartridges may be held by hand. In one embodiment, the cartridges are secured upon a support base, such as a metal LC-800 base.

In one embodiment, an insertion tool having an insertion fork may be utilized in conjunction with a needle driver having opposing clamping jaws that hold the insertion tool.

In one embodiment, an insertion tool may have a shaft with a distal end that includes an insertion fork. The shaft of the insertion tool may be straight, curved, or angled. In one embodiment, the shaft of the insertion tool is curved to mimic the configuration of a suture needle.

In one embodiment, because the insertion tool is held manually by a user, the insertion tool provides the user with more flexibility that enables mechanical fixation of the surgical fastener in a manner that is closer to that found when using sutures and suture needles.

In one embodiment, the insertion tools disclosed herein may be used for inserting surgical fasteners during open procedures such as open inguinal procedures, open ventral fixation procedures, and laparoscopic procedures.

In one embodiment, the length and geometry of the insertion tools may be modified to accommodate different surgical procedures.

In one embodiment, the surgical fasteners may have first and second legs having distal ends with respective insertion tips. In one embodiment, the legs may be curved for shallower implant fixation procedures.

In one embodiment, an insertion tool having an insertion fork may include a luer type connector connected to a proximal end of the insertion fork.

In one embodiment, the manual applicator system disclosed herein eliminates the need for more expensive, disposable gun-like applicator instruments that are used only once and then disposed.

In contrast to disposable gun-like applicator instruments that have a single type of fastener, the cartridge system disclosed herein provides more flexibility with respect to 1) using different sized surgical fasteners, 2) using surgical fasteners made of different materials, 3) having access to cartridges having different quantities of surgical fasteners, and 4) having different sized straps within a single cartridge.

The present invention provides many of the benefits found in gun-like applicator instruments without the cost of a pre-loaded device. Rather than requiring a complicated device and its components to be revised for each strap configuration, the present invention is able to easily accommodate different surgical fastener configurations and sizes by providing a simple cartridge system.

The manual insertion system disclosed herein provides a number of advantages over gun-like applicator systems that dispense a fastener each time a trigger is pulled. In one embodiment, the manually controlled insertion tool provides a user with a one-to-one tactile feel when inserting a surgical fastener into tissue. In one embodiment, the manually controlled insertion tool enables a user to manually control the amount of insertion force used to insert a surgical fastener into tissue. In one embodiment, the manually controlled system enables the user to push a surgical fastener further into tissue, if desired.

In one embodiment, an applicator system for implant fixation enables surgical fasteners to be inserted into tissue manually. In one embodiment, an applicator system uses a standard needle driver that holds an insertion tool with an insertion fork between the clamping jaws of the needle driver.

In one embodiment, the applicator system includes an insertion tool that is pen-like with either a disposable or a re-usable handles. In one embodiment, single-use insertion forks are attached to an end of the handles.

In one embodiment, the surgical fasteners are held in the cartridge via the geometry of the surgical fastener or by annealing the surgical fastener. In one embodiment, the cartridge includes a flexible element that retains the surgical fasteners in the cartridge until an insertion fork is inserted into the cartridge to compress the flexible element for releasing a single surgical fastener.

In one embodiment, an applicator system includes an insertion tool that has either a straight, curved, or angled insertion fork. In one embodiment, the insertion tool preferably has a curved shaft that mimics the shape of a curved suture needle.

In one embodiment, the length and geometry of the insertion tool may be modified to accommodate different surgical procedures and different body locations.

In one embodiment, an insertion tool preferably includes a protective outer sheath that covers the insertion fork during advancement to a surgical site, and that is retracted relative to the insertion fork to expose the insertion fork and enable the insertion fork to be utilized for inserting a surgical fastener into tissue.

In one embodiment, an applicator system preferably includes a base that holds one or more cartridges atop the base using tongue and groove features found on the base and the cartridges.

These and other preferred embodiments of the present disclosure will be described in more detail below.

### BRIEF DESCRIPTION OF THE DRAWING

FIGS. 1A, 1B, 1B-1, and 1C show an applicator system for inserting surgical fasteners, in accordance with one embodiment of the present disclosure.
FIGS. 2A-2G show a surgical fastener for securing an implant to tissue, in accordance with one embodiment of the present disclosure.
FIGS. 3A-3E show an insertion fork and a surgical fastener, in accordance with one embodiment of the present disclosure.
FIGS. 4A-4D show a surgical fastener loaded onto an insertion fork, in accordance with one embodiment of the present disclosure.
FIG. 5 shows a perspective view of a surgical fastener, in accordance with one embodiment of the present disclosure.
FIGS. 6A-6C show a surgical fastener and an insertion fork, in accordance with one embodiment of the present disclosure.
FIG. 7A shows an insertion tool having an insertion fork, in accordance with one embodiment of the present disclosure.
FIGS. 7B-7D show the insertion tool of FIG. 7A secured to a needle driver, in accordance with one embodiment of the present invention.
FIG. 7E shows the insertion tool and the needle driver of FIGS. 7B-7D with a surgical fastener loaded onto the insertion fork of the insertion tool.
FIGS. 8A-8B show an insertion tool having an insertion fork, in accordance with one embodiment of the present disclosure.
FIGS. 9A-9B show the insertion tool of FIGS. 8A-8B secured to a needle driver, in accordance with one embodiment of the present invention.
FIG. 9C shows a surgical fastener loaded onto the insertion fork of the insertion tool of FIG. 9B.
FIG. 10A shows an insertion tool having an insertion fork, in accordance with one embodiment of the present disclosure.
FIGS. 10B-10C show the insertion tool of FIG. 10A secured to a needle driver, in accordance with one embodiment of the present invention.
FIGS. 11A-11C show an insertion tool having an insertion fork, in accordance with one embodiment of the present invention.
FIGS. 12A-12B show an insertion tool having an insertion fork, in accordance with one embodiment of the present disclosure.
FIGS. 13A-13B show an insertion tool having an insertion fork, in accordance with one embodiment of the present invention.
FIGS. 14 shows an insertion tool having an insertion fork, in accordance with one embodiment of the present invention.
FIG. 15 show an insertion tool including a needle driver having an insertion fork secured to a distal end of the needle driver, in accordance with one embodiment of the present disclosure.
FIG. 16 shows an insertion fork having a stop flange, in accordance with one embodiment of the present disclosure.
FIG. 17 shows an insertion tool including an insertion fork and a luer fitting, in accordance with one embodiment of the present disclosure.
FIG. 18 shows an insertion tool including an insertion fork and a luer fitting having a needle driver adapter, in accordance with one embodiment of the present disclosure.
FIGS. 19A-19C shows an insertion tool including a protective sheath and an insertion fork retractable inside the protective sheath, in accordance with one embodiment of the present disclosure.
FIG. 20 shows an insertion tool including an elongated shaft having multiple curves and an insertion fork secured to the distal end of the elongated shaft, in accordance with one embodiment of the present disclosure.
FIGS. 21A-21B show a cartridge for surgical fasteners, in accordance with one embodiment of the present disclosure.
FIGS. 22A-22B show a cartridge for annealed surgical fasteners, in accordance with one embodiment of the present disclosure.
FIG. 23A shows a cartridge for surgical fasteners, the cartridge having a flexible member, in accordance with one embodiment of the present disclosure.
FIGS. 23B and 23B show perspective and end views of the flexible member shown in FIG. 23A.
FIGS. 24A-24C show a method of removing surgical fasteners from the cartridge shown in FIGS. 23A-23C, in accordance with one embodiment of the present disclosure.
FIGS. 25A-25B show a cartridge for surgical fasteners, in accordance with one embodiment of the present disclosure.
FIG. 26 shows a support base for cartridges that contain surgical fasteners, in accordance with one embodiment of the present disclosure.
FIGS. 27A-27C show a surgical fastener having curved legs, in accordance with one embodiment of the present disclosure.
FIG. 28 shows a method of using an insertion tool during an open inguinal procedure, in accordance with one embodiment of the present disclosure.
FIG. 29 shows a method of using an insertion tool during an open ventral fixation procedure, in accordance with one embodiment of the present disclosure.
FIG. 30 shows a method of using an insertion tool during a laparoscopic procedure, in accordance with one embodiment of the present disclosure.

### DETAILED DESCRIPTION

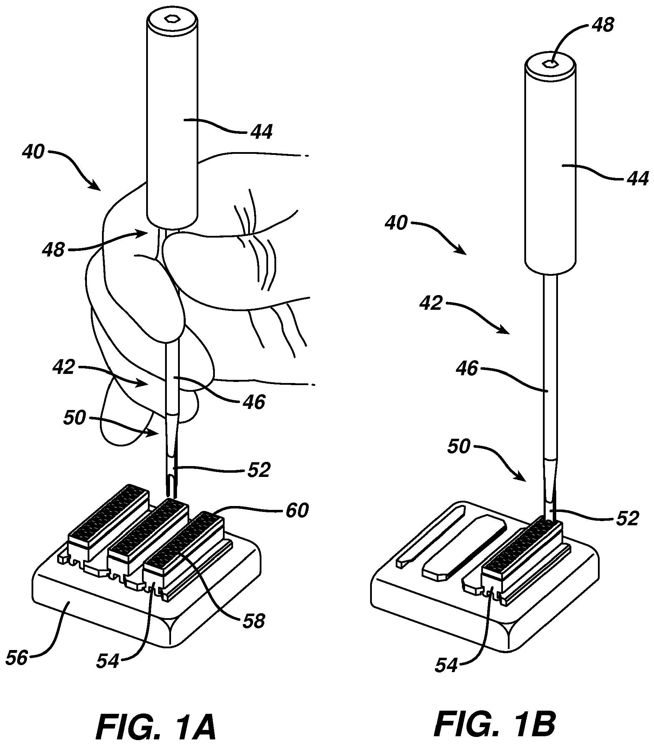

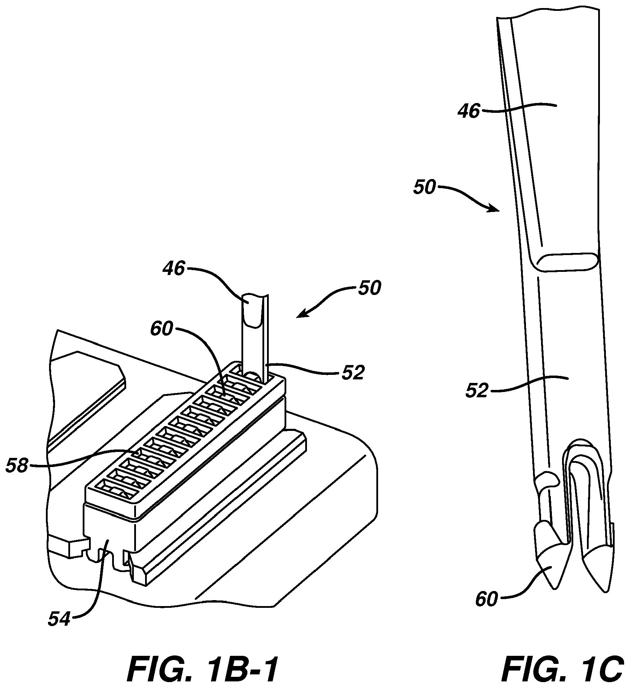

Referring to FIGS. 1A and 1B, in one embodiment, an applicator system 40 for dispensing surgical fasteners preferably includes an insertion tool 42 having a handle 44 and an elongated shaft 46 that projects distally from the handle 44. In one embodiment, the elongated shaft 46 has a proximal end 48 secured to the handle 44 and a distal end 50 that has an insertion fork 52 connected thereto. As will be described in more detail herein, the insertion fork 52 is utilized for secured thereto.

In one embodiment, the applicator system 40 includes one or more cartridges 54 that are mounted atop a support base 56 that holds the cartridges. In one embodiment, each cartridge 54 includes a plurality of slots 58 that are accessible at a top surface of the cartridge 54. A single surgical fastener 60 is disposed within each of the slots 58. In one embodiment, each of the cartridges 54 contains a plurality of surgical fasteners 60. The surgical fasteners in one cartridge may have the same properties (e.g., the same size), or the surgical fasteners in one cartridge may be divided into different sections having different properties (e.g., small, medium, and large sizes). In one embodiment, all of the surgical fasteners in a first cartridge may have a first property (e.g., small size), and all of the surgical fasteners in a second cartridge may have a second property (e.g., large size).

In one embodiment, the base 56 and the cartridges 54 may have tongue and groove features that are used for securing the cartridges atop the base 56. In one embodiment, the tongue and groove features are used for releasably securing the cartridges to the base, and for mixing and matching cartridges having surgical fasteners with different properties. The cartridges may be slid over a top surface of the base using the tongue and groove features, which then hold the cartridges in place atop the base.

Referring to FIGS. 1B and 1B∼1, in one embodiment, in order to load a surgical fasteners onto the distal end of the insertion tool 42, the fork 52 at the distal end 50 of the elongated shaft 46 is inserted into one of the slots 58 of a cartridge 54 for engaging the surgical fastener disposed within the slot. A single surgical fastener is loaded onto the distal end of the insertion tool 42 each time the fork is loaded into an elongated slot that contains a surgical fastener.

Referring to FIGS. 1B-1 and 1C, in one embodiment, when the surgical fastener 60 has been loaded onto the fork 52 at the distal end 50 of the elongated shaft 46, the fork 52 is withdrawn from the elongated slot 60 of the cartridge 54, whereupon the surgical fastener 60 remains secured to the fork 52. The fork 52 and the surgical fastener 60 loaded onto the fork will then be inserted into tissue for securing an implant, such as a surgical mesh, to the tissue. The inserted surgical fastener preferably engages the implant for securing the implant to the tissue. Once the surgical fastener is inserted into the tissue, the fork 52 is retracted and the surgical fastener remains disposed in the tissue.

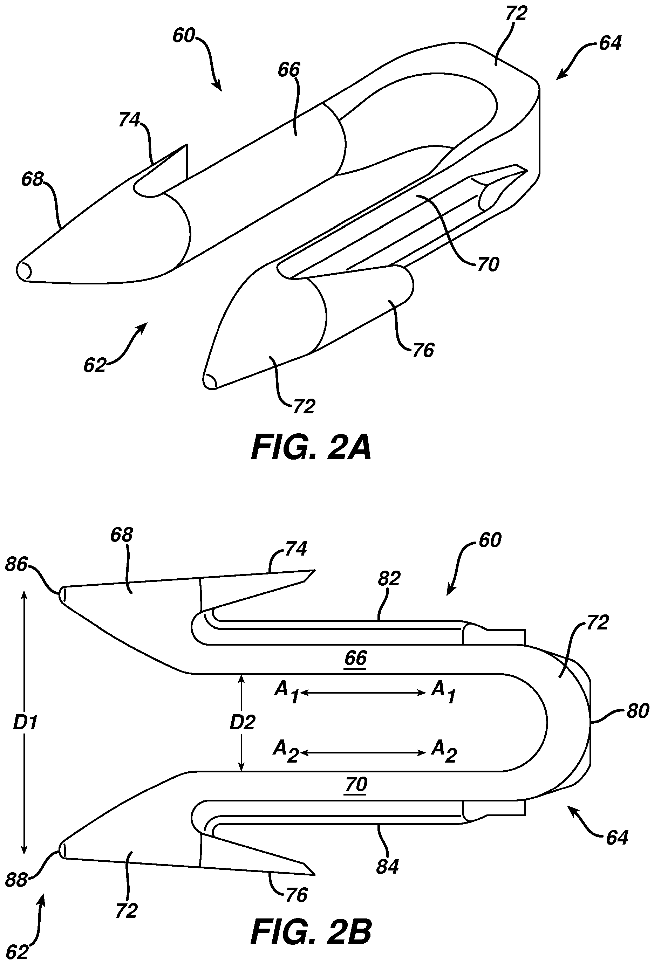

Referring to FIGS. 2A-2F, in one embodiment, the surgical fastener 60 desirably includes a leading or distal end 62 and a trailing or proximal end 64. The surgical fastener 60 preferably includes a first leg 66 having a first insertion tip 68 provided at a distal end of the first leg, and a second leg 70 having a second insertion tip 72 provided at a distal end of the second leg. In one embodiment, the cross-sectional dimension of each first and second leg 66, 70 diminishes when moving from the proximal ends toward the distal ends of the respective legs. The surgical fastener 60 preferably includes a bridge 72 adjacent the proximal end 64 of the surgical fastener that connects the proximal ends of the first and second legs 66, 78. In one embodiment, the bridge 72 may be positioned anywhere between the proximal and distal ends of the surgical fastener so long as it interconnects the first and second legs. The surgical fastener 60 preferably includes at least one first barb 74 projecting rearwardly from the first insertion tip 68 and at least one second barb 76 projecting rearwardly from the second insertion tip 72. Although only one barb is shown on each leg, other surgical fasteners may have multiple barbs on each leg or insertion tip. The barbs may extend away from one another (e.g., outwardly directed barbs), may extend toward one another (e.g., inwardly directed barbs), or may extend in the same direction (e.g., extending in the same direction from the tops or bottoms of the legs). The first and second insertion tips 68, 72 may be conical in shape. The respective insertion tips 68, 72 preferably have distal-most points 86, 88 that define the leading ends of the first and second legs 66, 70. The distal-most points 86, 88 may be sharp, blunt, or obtuse. In one embodiment, the first and second insertion tips 68, 72 have blunt distal piercing points 86, 88. The blunt points enable the surgical fastener 60 to penetrate tissue while minimizing unwanted penetration into the hand of an operator.

In one embodiment, the first and second insertion tips 68, 72 are preferably skewed with respect to longitudinal axes of the respective first and second legs 66, 70. In one embodiment, the insertion tips are skewed outwardly with respect to the longitudinal axes of the first and second legs.

Referring to FIG. 2B, in one embodiment, the bridge 72 preferably includes a concave inner surface 78 facing toward the distal end 62 of the surgical fastener 60 and a convex outer surface 80 facing away from the distal end 62 of the surgical fastener. The first leg 66 preferably has an outer wall having a first rib 82 that extends along a longitudinal axis A₁ of the first leg. The second leg 70 preferably has an outer wall having a second rib 84 that extends along the longitudinal axis A₂ of the second leg. In one embodiment, the distance D₁ between the respective distal-most points 86, 88 at the distal ends of the first and second insertion tips 68, 72 is preferably greater than the distance D₂ between the opposing surfaces of the first and second legs 66, 70. The wider relative distance between the distal piercing points of the first and second tips 68, 72 preferably ensures that the surgical fastener will engage strands on a porous prosthetic device, such as the strands of a surgical mesh. In one embodiment, the outwardly skewed insertion tips 68, 72 enhance the likelihood of capturing surgical mesh fibers between the first and second legs without increasing the lateral distance D₂ between each leg.

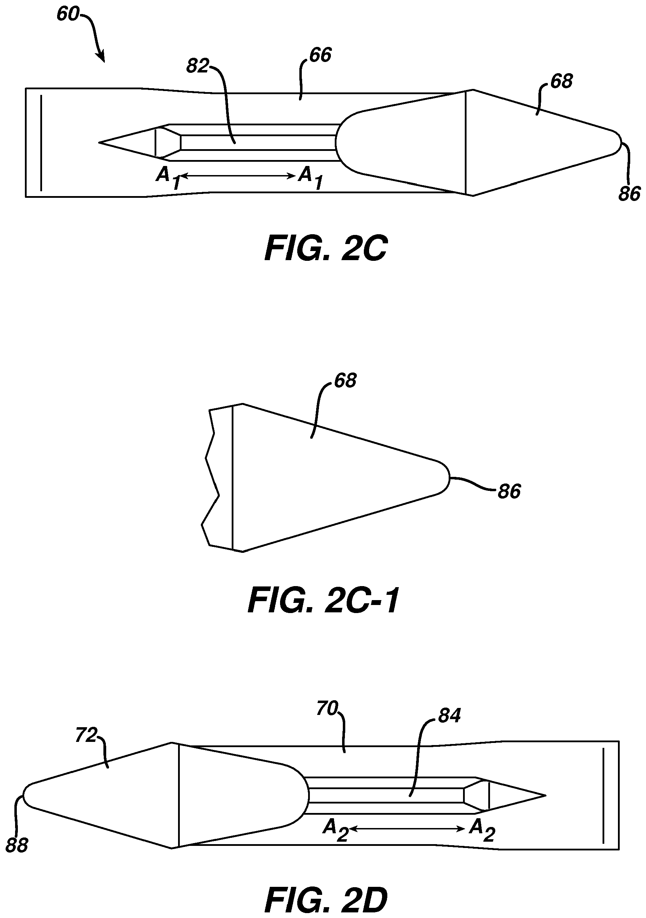

Referring to FIG. 2C, in one embodiment, the first leg 66 of the surgical fastener 60 has the first rib 82 extending along the longitudinal axis A₁ of the first leg. When viewed from the side as shown in FIG. 2C, the first rib 82 is preferably in substantial alignment with the distal-most point 86 at the distal end of the first insertion tip 68.

FIG. 2C-1 shows an enlarged view of the first insertion tip 68 including the distal-most point 86. In one embodiment, the distal-most point 86 enables the distal end of the surgical fastener to penetrate tissue while minimizing unwanted penetration into the hand of an operator.

Referring to FIG. 2D, in one embodiment, the second leg 70 of the surgical fastener 60 has the second rib 84 extending along the longitudinal axis A₂ of the second leg 70. When viewed from the side as shown in FIG. 2D, the second rib 84 is preferably aligned with the distal-most point 88 at the distal end of the second insertion tip 72.

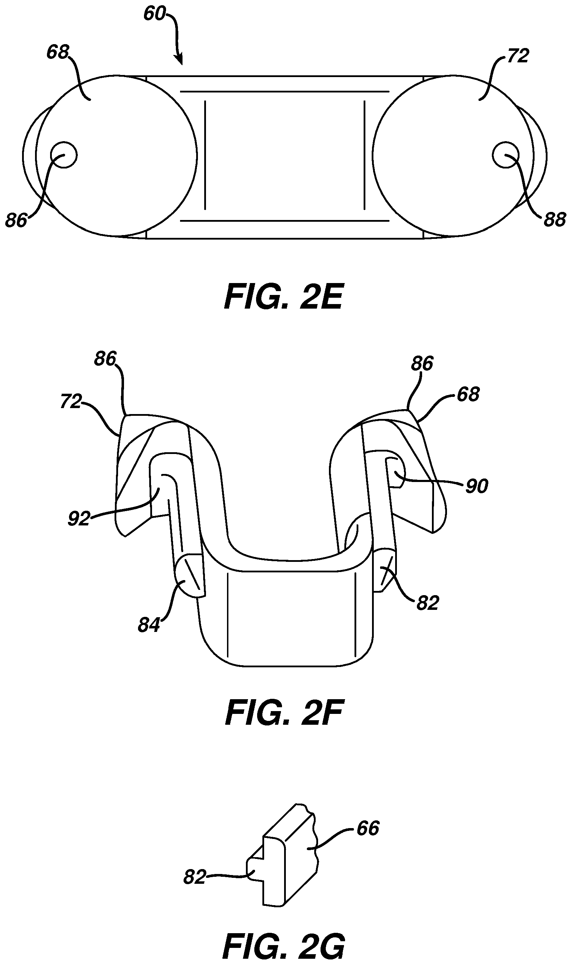

Referring to FIG. 2E, in one embodiment, the first and second insertion tips 68, 72 including the distal-most points 86, 88, respectively, are preferably skewed outwardly from a center of the surgical fastener 232. In one embodiment, the first and second insertion tips 68, 70 are preferably asymmetrical and are configured to extend outwardly from the center of the surgical fastener 60.

Referring to FIG 2F, in one embodiment, the rear face (i.e., the proximal end) of the first insertion tip 68 preferably includes a first insertion tool seating surface 90 adapted to receive a distal end of a first tine of the insertion fork 52 (FIG. 1C). The rear face of the second insertion tip 72 preferably includes a second insertion tool seating surface 92 adapted to receive a distal end of a second tine of the insertion fork 52 (FIG. 1C). In one embodiment, the first and second insertion tool seating surfaces preferably define convexly curved surfaces. In one embodiment, the insertion tool seating surfaces 90, 92 are preferably substantially aligned with the distal-most points 86, 88 of the first and second insertion tips 68, 72. The distal ends of the tines of the insertion fork may have surfaces that conform to surfaces of the respective insertion tool seating surfaces 90, 92.

Referring to FIG. 2G, in one embodiment, the first leg 66 has an inner face that is rounded and an outer face that is squared-off. The first rib 82 preferably extends along the length of the first leg 66. The second leg 70 desirably has similar features as shown for the first leg in FIG. 2G. Although the present disclosure is not limited by any particular theory of operation, it is believed that providing a surgical fastener with legs such as the leg shown in FIG. 2G increases the strength of the surgical fastener by increasing the section modulus. Providing legs having a cross-section with an inner rounded-off surface and an outer squared-off surface also preferably increases the force required to pull the surgical fastener out of tissue once the fastener has been inserted into tissue.

In one embodiment, the surgical fastener may be made of absorbable and/or non-absorbable materials. Preferred absorbable materials include PDS, PDS/lactide-glycolide blends, PLA, etc. In one embodiment, each surgical fastener is sized to fit inside of a 5 mm outer diameter tube (typically trocar cannula dimension). The surgical fastener is fabricated by molding, however, with small modifications, other processes such as casting, stamping, and machining may be used. In one embodiment, the surgical fasteners may be extruded into a general shape, and then formed.

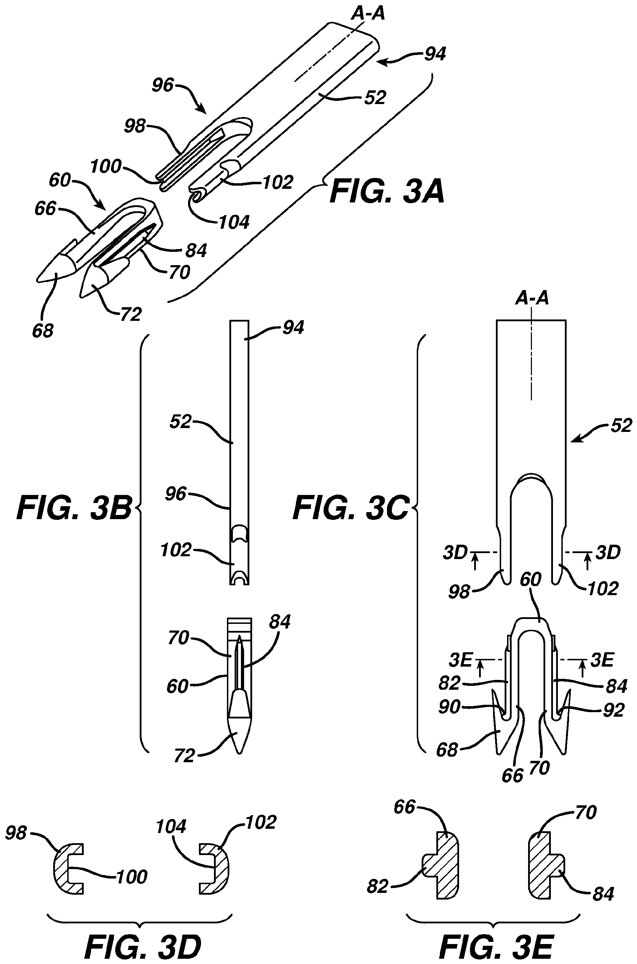

Referring to FIGS. 3A-3E, in one embodiment, the surgical fastener 60 described above in FIGS. 2A-2G is loaded onto the insertion fork 52 at the distal end of the elongated shaft 46 (FIG. 1A) of the insertion tool. In one embodiment, the insertion fork 52 preferably includes a proximal end 94 that is connected to a distal end of the elongated shaft of the insertion tool and a distal end 96 adapted to engage one or more surfaces of the surgical fastener 60. In one embodiment, the distal end 96 of the insertion fork 52 preferably includes a first tine 98 having a first inner groove 100 formed therein, and a second tine 102 having a second inner groove 104 formed therein. In one embodiment, the inner grooves 100, 104 on the tines preferably oppose one another and extend along axes that are parallel with the longitudinal axis A-A of the fork 52 (FIG. 3a). In one embodiment, the inner grooves 100, 104 preferably have a C-shaped cross-section that generally conforms to the configuration of the ribs 82, 84 on the first and second legs 66, 70 of the surgical fastener 60.

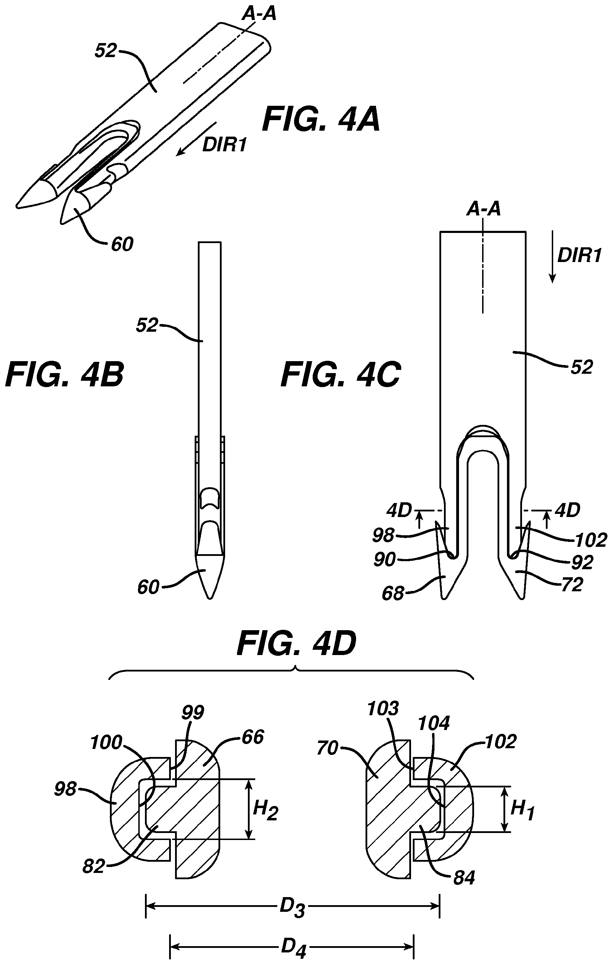

Referring to FIGS. 4A-4D, in one embodiment, the surgical fastener 60 is loaded onto the fork 52 by sliding the opposing inner grooves 100, 104 of the first and second tines 98, 102 over the ribs 82, 84 on the respective first and second legs 66, 70 of the surgical fastener. The engagement of the inner grooves 100, 104 with the ribs 82, 84 preferably aligns the surgical fastener 60 with the distal end 96 of the insertion fork 52, and stabilizes the surgical fastener during implantation in tissue. In one embodiment, the distal-most tips of the first and second tines 98, 102 are advanced until they abut against the insertion tool seating surfaces 90, 92 provided at the proximal end of the first and second insertion tips 68, 72. In one embodiment, the fork 52 is advanced along the axis A-A in the direction designated DIR1 (FIG. 4A) until the distal ends of the tines 98, 102 abut against the insertion tool seating surfaces 90, 92 (FIG. 4C) provided at the proximal ends of the respective first and second insertion tips 68, 72.

Referring to FIG. 4C, in one embodiment, the engagement of the distal end of the first tine 98 with the first insertion tool seating surface 90 preferably provides a first point of contact between the fork 52 and the surgical fastener 60. The engagement of the distal end of the second tine 102 with the second insertion tool seating surface 92 provides a second point of contact between the fork 52 and the surgical fastener 60. In one embodiment, the insertion fork 52 also engages the rear face 80 of the bridge 72 of the surgical fastener 60 to provide a third point of contact between the fork and the surgical fastener for driving the surgical fastener into tissue.

Referring to FIG. 4D, in one embodiment, the first rib 82 on the first leg 66 is captured within the concave groove 100 of the first tine 98, and the second rib 84 on the second leg 70 is captured within the concave groove 104 of the second tine 102. In one embodiment, the distance D₃ between the outer faces of the first and second ribs 82, 84 is preferably greater than the distance D₄ between the opposing faces 99, 103 of the respective first and second tines 98, 102 so that the first and second legs 66, 70 are reliably secured between the tines 98, 102 of the insertion fork 52. The height H₁ of the ribs 82, 84 is slightly less than the height H₂ of the concave grooves 100, 104 so that the tines 98, 102 slide loosely over the ribs 82, 84.

Although the present disclosure is not limited by any particular theory of operation, it is believed that providing an insertion fork with grooved tines that engage ribs on outer surfaces of the legs of a surgical fastener will enhance stability and control of the surgical fastener when dispensing the surgical fastener from the distal end of the applicator instrument. In addition, the insertion force is provided closer to the distal end of the surgical fastener and not only at the proximal end of the surgical fastener as is the case with prior art systems. This feature (i.e. providing insertion force on the surgical fastener near the leading end of the fastener) may enable smaller and/or lower profile surgical fasteners to be used. The insertion fork also provides additional insertion force where the fork engages the bridge of the surgical fastener at the trailing end of the fastener.

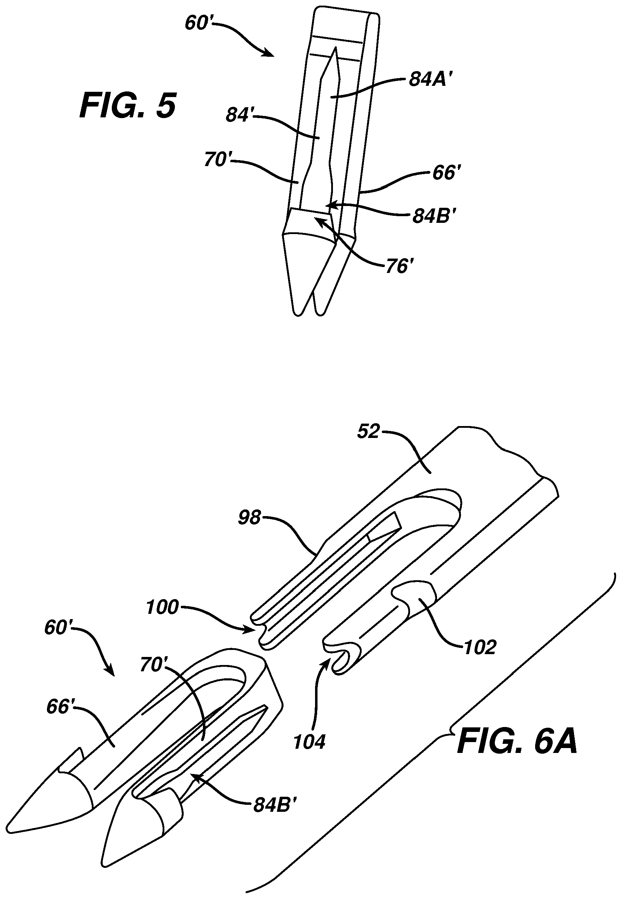

In the embodiments shown in FIGS. 2A-2G, the ribs 82, 84 on the surgical fasteners 60 have a width that remains constant between the proximal and distal ends thereof. In one embodiment, a surgical fastener may have ribs having widths that change between the proximal and distal ends of the ribs. FIGS. 5 and 6A-6C show one such embodiment.

Referring to FIG. 5, in one embodiment, a surgical fastener 60' includes a first leg 66' and a second leg 70'. The surgical fastener 60' is generally similar in structure and configuration to that shown in FIGS. 2A-2G, except for the ribs that extend along the legs. Referring to FIG. 5, in one embodiment, the second leg 70' of the surgical fastener includes a rib 84' that extends along the length of the second leg. Referring to FIGS. 5 and 6A, the second rib 84' has a narrower proximal section 84A' and a wider distal section 84B', the latter section being configured to form an interference fit with the concave groove 104 provided on the second tine 102 of the insertion fork 52. Although not shown, the first leg 66' preferably has a rib that also has a narrower proximal section and a wider distal section, whereby the latter section is designed to form an interference fit with the concave groove 100 of the first tine 98.

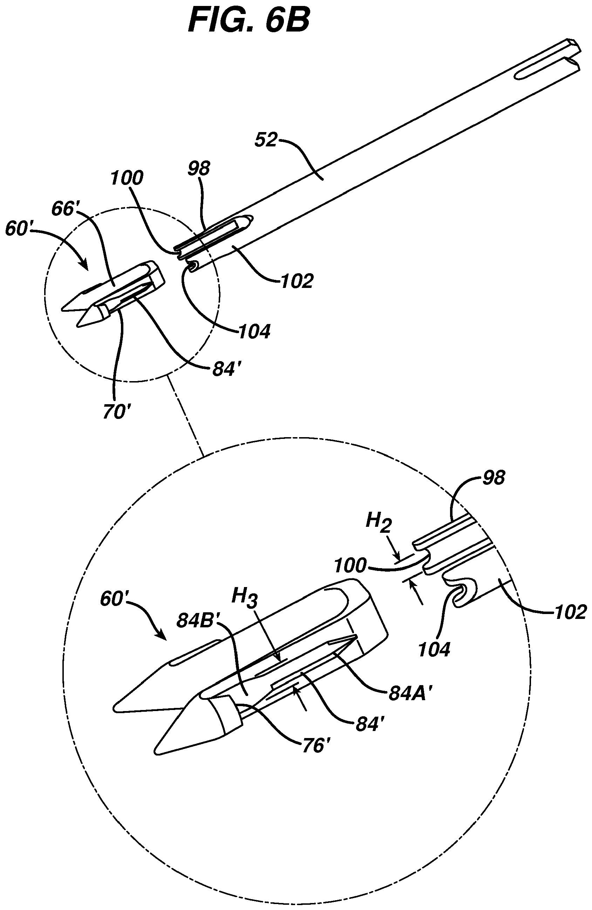

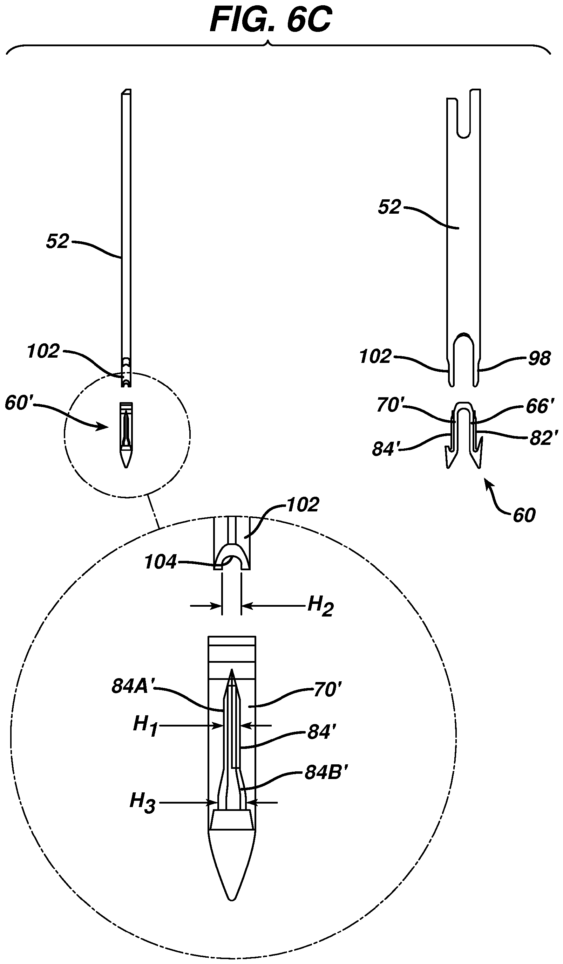

Referring to FIGS. 6B and 6C, the elongated channels 100, 104 of the first and second tines 98, 102 have a height H₂ that is slightly larger than the height H₁ (FIG. 6C) of the proximal section 84A' of the rib 84'. The wider distal section 84B' of the rib 84' has a height H₃ that is greater than the height H₂ of the elongated channels 100, 104. When the surgical fastener 60' is loaded onto the tines 98, 102 of the insertion fork 52, an interference fit is formed between the wider distal sections of the ribs 82', 84' and the elongated C-shaped channels 100, 104 of the tines 98, 102. The interference fit between the wider distal sections of the ribs 82', 84' and the C-shaped channel is strong enough to hold the surgical fastener 60' on the insertion fork after initial loading. After insertion of the surgical fastener into tissue, however, the interference fit is not sufficiently strong to prevent the surgical fastener from being pulled off the fork by the barbs 74', 76' biting into the tissue.

In one embodiment, only one of the first and second ribs 82', 84' may have a narrower proximal section and a wider distal section. In one embodiment, both the first and second legs have respective first and second ribs with narrower proximal sections and wider distal sections. In FIGS. 6A, 6B, and 6C, the barb 76' on the second leg 70' has been cut away to provide clarity of the wider distal section 84B' of the second rib 84'.

The insertion forks and surgical fasteners disclosed herein may be incorporated into a wide variety of surgical fastener insertion tools having various features. The applicator instruments may be stand-alone tools or may be combined with other well-known surgical tools.

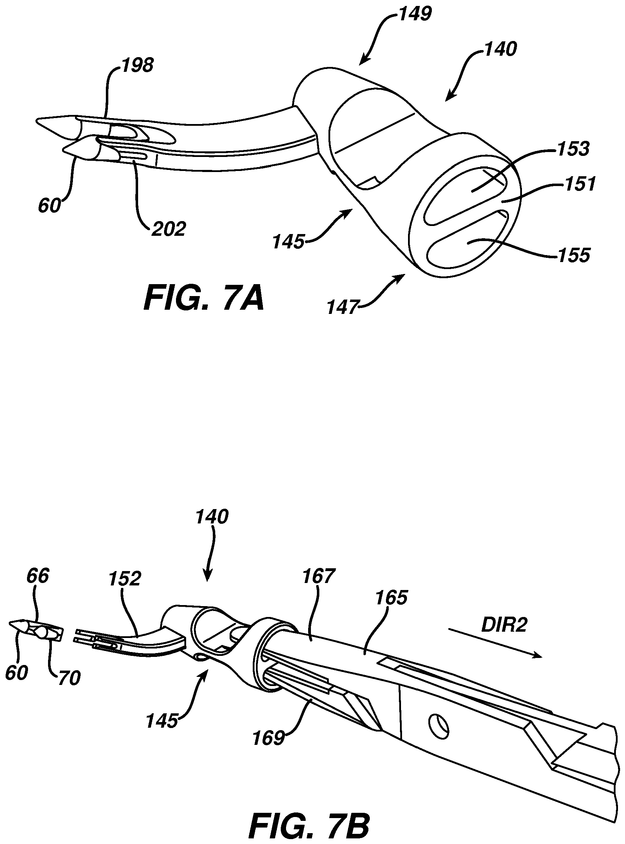

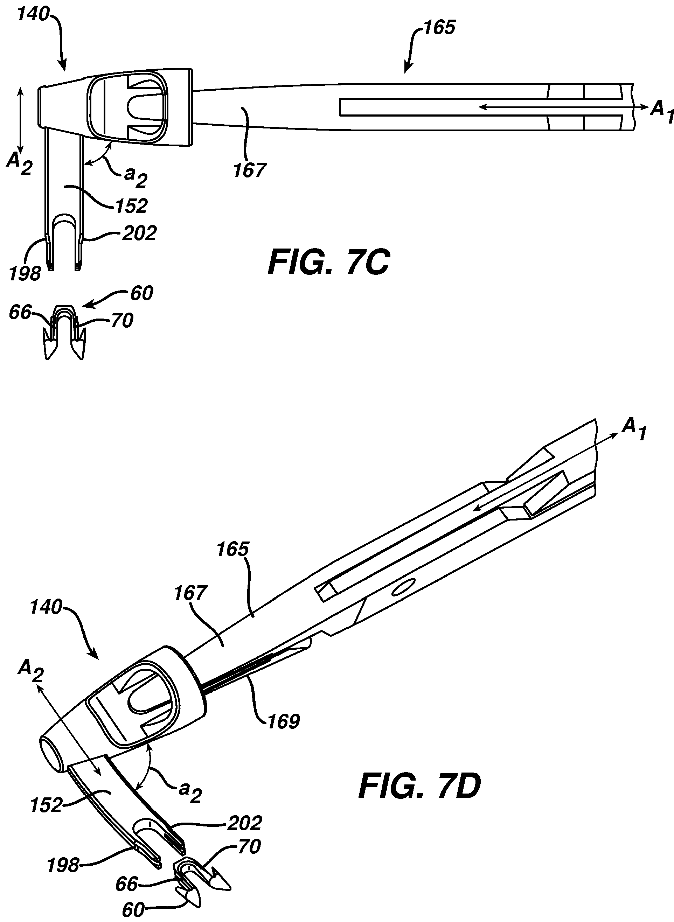

Referring to FIG. 7A, in one embodiment, a surgical fastener insertion tool 140 preferably includes a base 145 having a proximal end 147 and a distal end 149. The proximal end 147 of the base 145 has a proximal end face 151 with a first opening 153 at an upper end of the proximal end face 151 and a second opening 155 at the lower end of the proximal end face 151. In one embodiment, the first and second openings 153, 155 formed in the proximal end face 151 of the body 145 are preferably tapered openings that narrow between the proximal end 147 and the distal end 149 of the body 145.

In one embodiment, the insertion tool 140 preferably includes an insertion fork 152 secured to the distal end 149 of the body 145. The insertion fork 152 preferably extends laterally relative to the longitudinal axis A₁ of the body 145. The insertion fork 152 includes a first tine 198 and a second tine 202 that oppose one another and that are adapted for securing a surgical fastener 60 therebetween. The insertion fork 152 may have one or more of the features disclosed in the embodiment shown in FIGS. 3A-3E and 4A-4D.

Referring to FIGS. 7B-7D, in one embodiment, the insertion tool 140 may be secured to the distal end of a needle driver 165. In one embodiment, the needle driver 165 has a first jaw 167 that is inserted into the first opening 153 of the body 145, and a second jaw 169 that is inserted into the second opening 155 of the body 145. The first and second openings 153, 155 are preferably tapered to form a snug fit between the body 145 and the jaws 167, 169 as the body 145 is pressed onto the jaws in a proximal direction designed DIR2 (FIG. 7B).

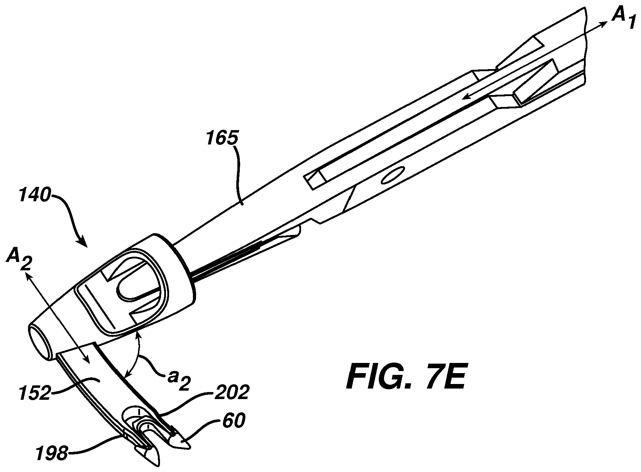

FIGS. 7B-7D show the insertion tool 140 after it has been mounted onto the jaws 167, 169 of the needle driver 165. In order to load a surgical fastener 60 onto the fork 152, the tines 198, 202 on the fork 152 are preferably aligned with the first and second legs 66, 70 of the surgical fastener 60. FIG. 7E shows the surgical fastener 60 secured between the tines 198, 202 of the insertion fork 152.

Referring to FIGS. 7C-7E, in one embodiment, the fork 152 extends along an axis A₂ that defines an angle α₁ with the longitudinal axis A₁ of the needle driver 165. In one embodiment, the angle α₁ is about 90 degrees. In one embodiment, the angle may be an acute angle or an obtuse angle. In one embodiment, the fork 152 has a curved shaft that mimics the shape and configuration of a curved suture needle.

Although the present disclosure is not limited by any particular theory of operation, it is believed that the embodiments shown in FIGS. 7A-7E provides an insertion tool having the look, feel and operational characteristics that are similar to a suture needle, which will add a level of comfort, confidence, and efficiency for surgical personnel that are very familiar with using convention, curved suture needles.

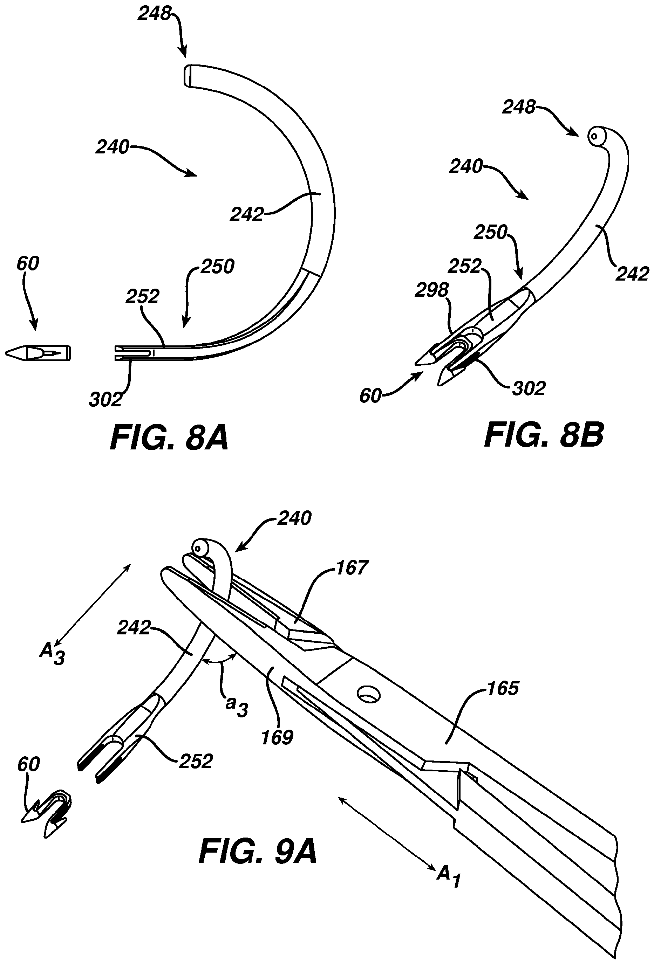

Referring to FIGS. 8A and 8B, in one embodiment, an insertion tool 240 used for inserting a surgical fastener in tissue includes an elongated shaft 242 having a proximal end 248 and a distal end 250 with an insertion fork 252 attached to the distal end 250 of the elongated shaft 242. In one embodiment, the elongated shaft 242 is preferably curved between the proximal end 248 and distal end 250 thereof. In one embodiment, the curved elongated shaft 242 defines a C-shaped curve.

The insertion tool 240 includes the insertion fork 252 having a first tine 298 and a second tine 302 opposing the first tine. The insertion fork 252 may have one or more of the features disclosed in the embodiment shown in FIGS. 3A-3E and 4A-4D. The insertion fork 252 is adapted to secure the surgical fasteners 60 (FIGS. 2A-2G), 60' (FIGS. 5, 6A-6C) shown and described herein.

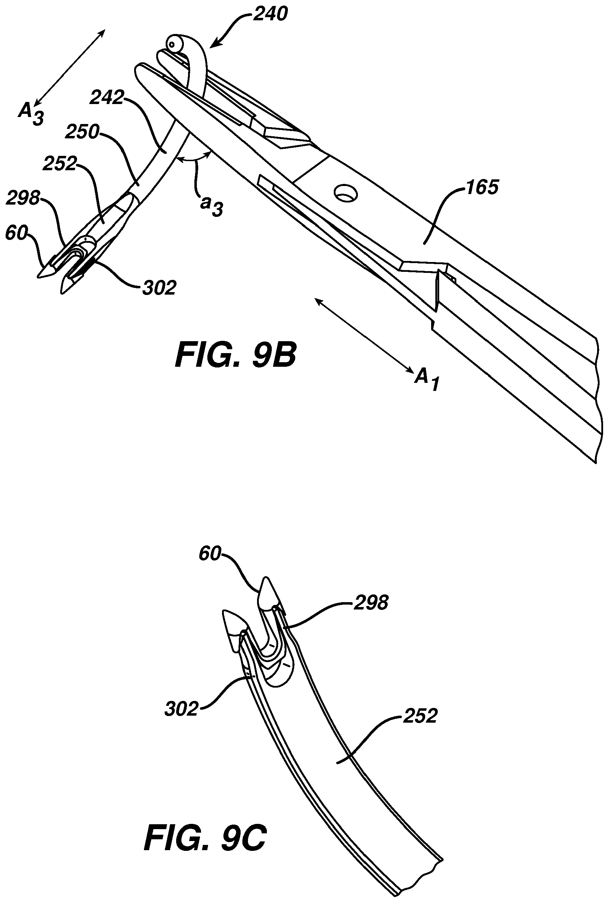

Referring to FIGS. 9A-9C, in one embodiment, the elongated shaft 242 of the surgical fastener insertion tool 240 is grasped between the clamping jaws 167, 169 of the needle driver 165. A surgical fastener 60 may be loaded onto the fork 252 at the distal end of the elongated shaft 242 of the insertion tool 240. FIG. 9A shows the fork 252 aligned with the proximal end of the surgical fastener 60. FIGS. 9B and 9C show the surgical fastener secured between the first and second tines 298, 302 of the fork 252.

Once the surgical fastener 60 is loaded onto the fork 252, the elongated shaft 242 of the insertion tool 240 is clamped between the jaws 167, 169, with the insertion fork 252 extending in a generally perpendicular orientation relative to the longitudinal axis A₁ of the needle driver 165. The tines 298, 302 of the insertion fork 252 are advanced into tissue for inserting the surgical fastener 60 into the tissue. The curved elongated shaft 242 of the insertion tool 240 mimics the look, feel, and operational characteristics of a curved suture needle.

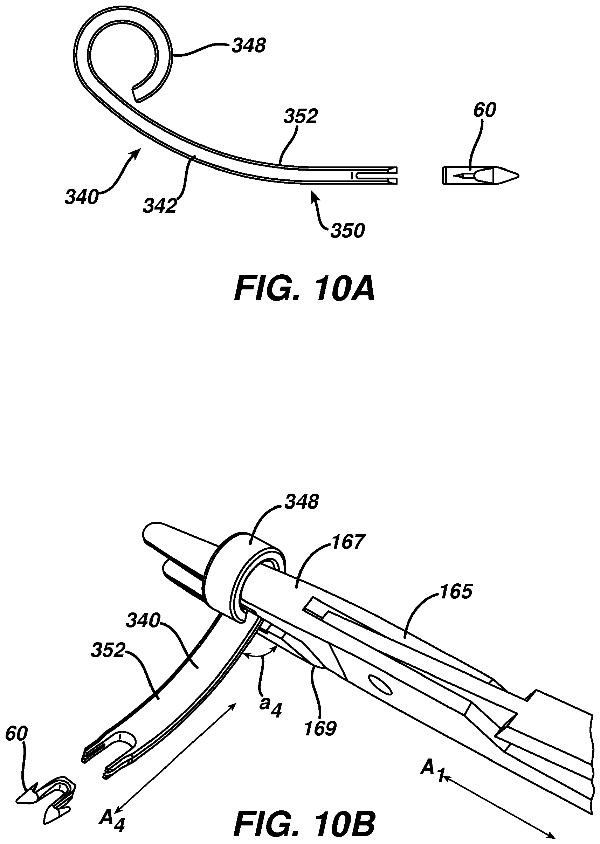

Referring to FIGS. 10A-10C, in one embodiment, an insertion tool 340 for surgical fasteners preferably includes an elongated shaft 342 having a proximal end 348 with a loop and a distal end 350 having an insertion fork 353 secured thereto. The fork at the distal end of the insertion tool 340 is adapted to secure a surgical fastener 60 to the insertion tool 340. The insertion fork 352 may have one or more of the features disclosed in the embodiment shown in FIGS. 3A-3E and 4A-4D. The insertion fork 352 is preferably adapted to secure any of the surgical fasteners 60 (FIGS. 2A-2G), 60' (FIGS. 5, 6A-6C) shown and described herein.

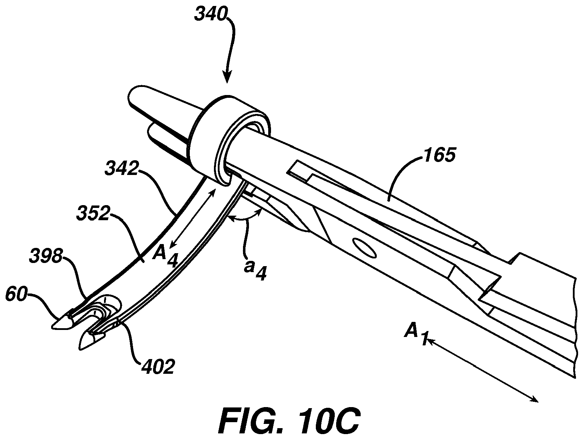

Referring to FIGS. 10B and 10C, in one embodiment, the first jaw 167 of the needle driver 165 is inserted into the loop at the proximal end 348 of the insertion tool 340. The second jaw 169 of the needle driver 165 is clamped onto the outside of the loop to secure the proximal end 348 of the insertion tool 340 between the first and second jaws 167, 169 of the needle driver 165. The fork 352 at the distal end of the insertion tool 340 is utilized to secure a surgical fastener 60 onto the distal end of the insertion tool. FIG. 10C shows the surgical fastener 60 after it has been secured between tines 398, 402 of the fork 352 of the insertion tool 340. The elongated shaft 342 of the insertion tool 340 extends laterally to the side relative to the longitudinal axis A₁ of the needle driver 165. As a result of this configuration, the use of the insertion tool 340 mimics that of a suture needle, which will provide a level of familiarity and comfort to surgical personnel. In one embodiment, the elongated shaft 342 is perpendicular to the longitudinal axis A1 of the needle driver. In one embodiment, the elongated shaft 342 may form an acute angle or obtuse angle with the longitudinal axis A₁ of the needle driver 165.

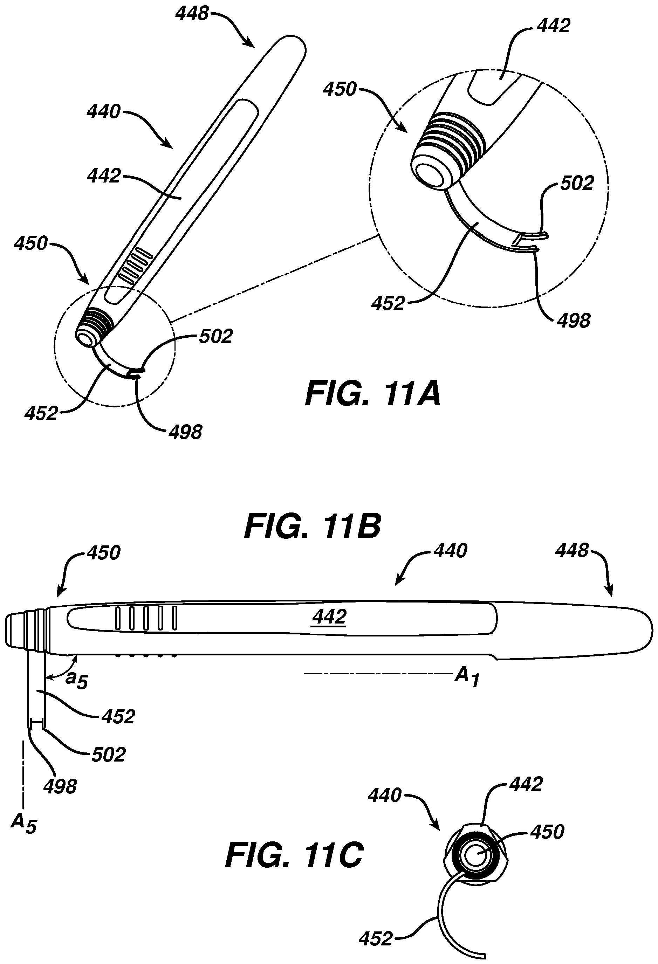

Referring to FIGS. 11A-11C, in one embodiment, an insertion tool 440 includes a handle 442 having a proximal end 448 and a distal end 450. The insertion tool 440 preferably includes an insertion fork 452 that is secured to the distal end of the handle. The insertion fork 452 preferably has tines 498, 502 that oppose one another and that are utilized for loading a surgical fastener onto the fork. In one embodiment, the insertion fork 452 has a curved shaft that mimics the shape and configuration of a suture needle. The insertion fork 352 may have one or more of the features disclosed in the embodiment shown in FIGS. 3A-3E and 4A-4D. The insertion fork 352 is preferably adapted to secure any of the surgical fasteners 60 (FIGS. 2A-2G), 60' (FIGS. 5, 6A-6C) shown and described herein.

In one embodiment, the insertion fork 452 extends along an axis A₅ that is perpendicular to the longitudinal axis A₁ of the handle 442. In one embodiment, the insertion fork 452 may extend along an axis that forms an acute or obtuse angle with the longitudinal axis of the handle 442.

In one embodiment, the insertion fork 452 is permanently secured to the distal end 450 of the handle 442. In one embodiment, the tines 498, 452 at the distal end of the fork 452 are inserted into a slot of a cartridge to engage a single surgical fastener between the opposing tines 498, 452. The handle 442 is grasped by surgical personnel and the use of the insertion tool 440 mimics that of a suture needle for inserting the surgical fastener (not shown) into tissue.

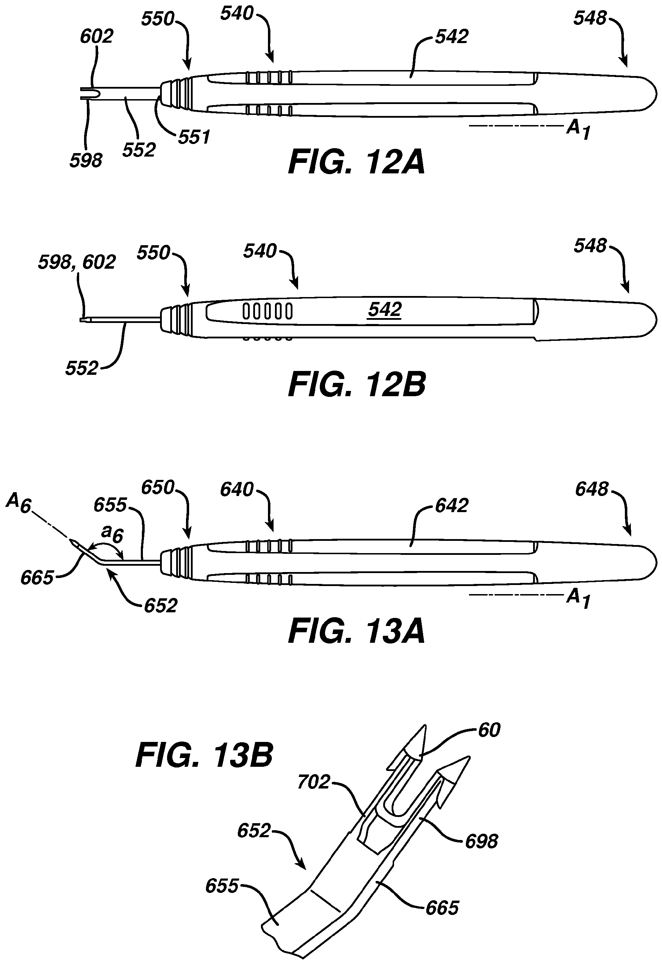

Referring to FIGS. 12A and 12B, in one embodiment, an insertion tool 540 includes a handle 542 having a proximal end 548 and a distal end 550. The distal end 550 of the handle 542 includes an axial opening 551 that is adapted to receive a proximal end of an insertion fork 552 having opposing tines 598, 602. The fork 452 has an elongated shaft that extends along the longitudinal axis A₁ of the handle 542. In one embodiment, the fork 452 may be removed after use and replaced with another fork having different features. In one embodiment, instead of using a straight fork, the insertion fork may have an angle or curve formed along the length thereof.

Referring to FIGS. 13A and 13B, in one embodiment, an insertion tool 640 for a surgical fastener preferably includes a handle 642 having a proximal end 648 and a distal end 650. The insertion tool desirably has an insertion fork 652 that is secured to the distal end 650 of the handle 642. In one embodiment, the insertion fork 652 has a proximal section 655 that extends along the longitudinal axis A₁ of the handle 642 and a distal section 665 that extends along an axis A₆ that defines an angle with the proximal section 655. In one embodiment, the angle between the distal section 665 and the proximal section 655 of the insertion fork 652 is approximately 20-40" and more preferably about 30°. The tines 698, 702 at the distal end of the fork 652 are utilized for loading the surgical fastener 60 onto the fork 652.

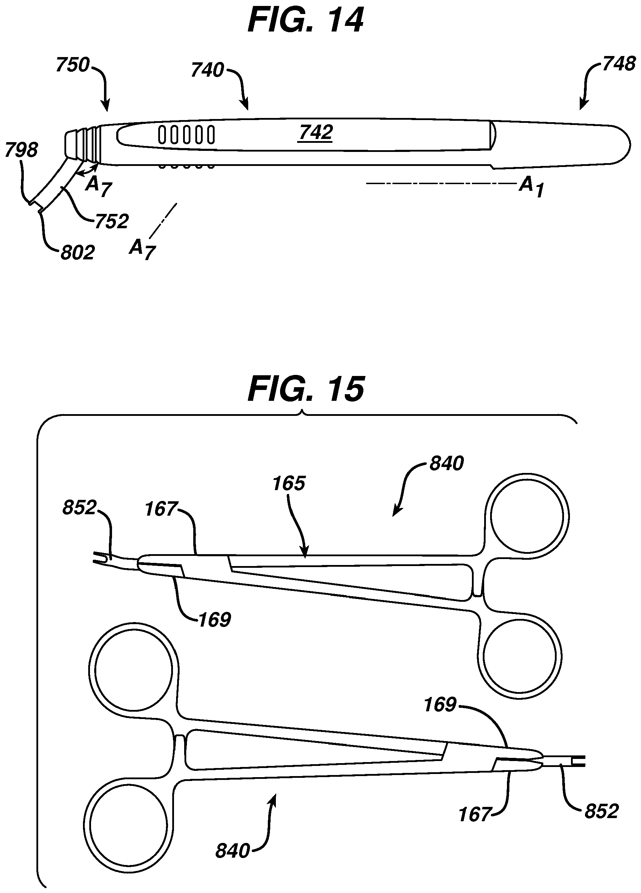

Referring to FIG. 14, in one embodiment, a surgical fastener insertion tool 740 preferably includes a handle 742 having a proximal end 748 and a distal end 750 remote therefrom. An insertion fork 752 is secured to the distal end 750 of the handle 742. The insertion fork has opposing tines 798, 802 adapted to engage a surgical fastener. The insertion fork 752 has an elongated shaft that extends along an axis A₇ that defines an angle designated α₇ with the longitudinal axis A₁ of the handle 742.

Referring to FIG. 15, in one embodiment, an insertion tool 840 for inserting surgical fasteners into tissue includes a needle driver 165 having opposing first and second clamping jaws 167, 169. An insertion fork 852, which is preferably similar to that disclosed herein, is held between the opposing clamping jaws 167, 169 of the needle driver 165. The fork 852 may be held between the opposing clamping halves or may be removably secured to one or more of the clamping jaws 167, 169. As described above, the fork 852 is utilized for loading a surgical fastener onto the distal end of the insertion tool 840.

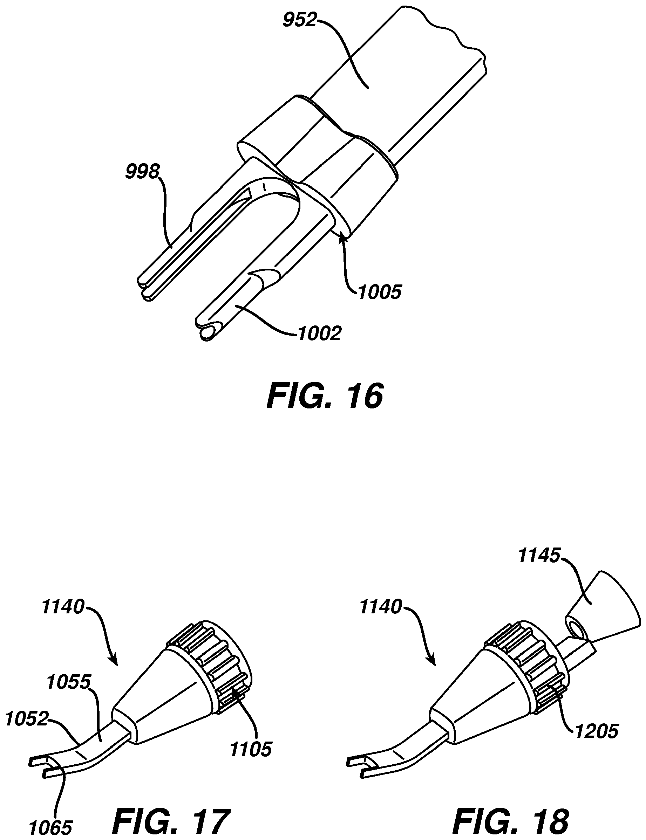

Referring to FIG. 16, in one embodiment, an insertion fork 952 has a distal end including tines 998, 1002. The insertion fork 952 may be utilized for loading surgical fasteners onto the fork and inserting the surgical fasteners into tissue. The insertion fork 952 preferably includes a stop 1005 that is proximal to the proximal ends of the tines 998, 1002. The stop preferably prevents over insertion of the fork 952 into tissue.

Referring to FIG. 17, in one embodiment, a surgical fastener insertion tool 1040 preferably includes an insertion fork 1052 connected with a luer fitting 1005. The luer fitting is adapted to secure the insertion fork to a medical device that may be coupled with the luer fitting 1105. In one embodiment, the insertion fork 1052 has a proximal section 1055, coupled with the luer fitting 1105, that extends along a first axis A₁, and a distal section 1065 that extends along an axis designated A₈ that defines an angle α₈ relative to the axis A₁ of the proximal section 1055. In one embodiment, the angle as is an obtuse angle. In one embodiment, the fork connected with the luer fitting may be straight along the entire length of the fork.

Referring to FIG. 18, in one embodiment, an insertion tool 1140 is generally similar to the embodiment described above in FIG. 17, however, the luer fitting 1205 preferably includes an adapter 1145 that may be used for connecting the luer fitting 1205 to the clamping jaws at the distal end of a needle colder.

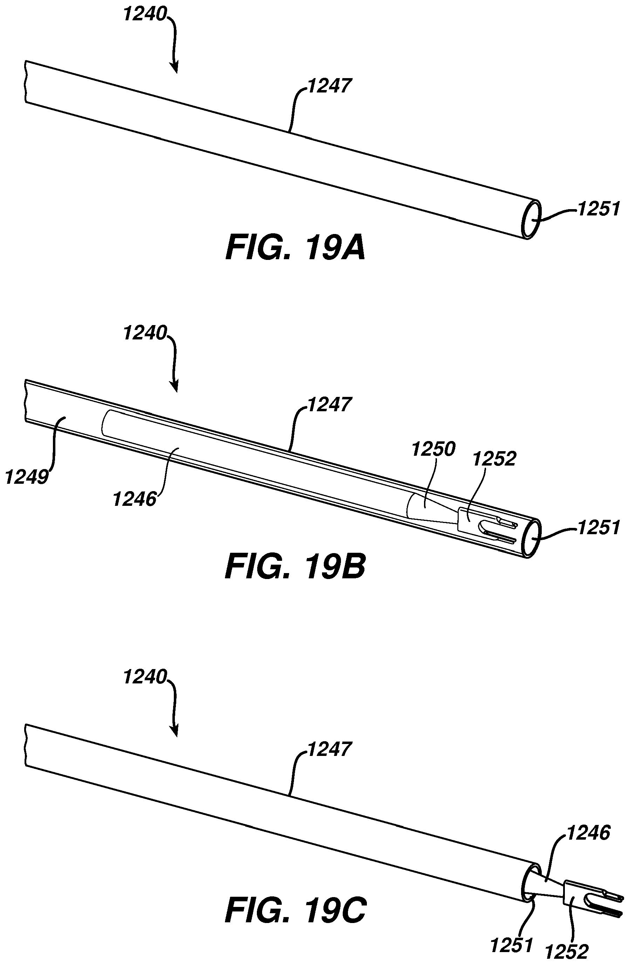

Referring to FIGS. 19A-19C, in one embodiment, an insertion tool 1240 for inserting surgical fasteners in tissue preferably includes an outer sheath 1247 having an elongated conduit 1249 and a distal end opening 1251 located at the distal end of the outer sheath 1247. The insertion tool 1240 preferably includes an elongated shaft 1246 having a distal end 1250 with an insertion fork 1252 secured to the distal end 1250 of the elongated shaft 1246.

In one embodiment, the elongated shaft 1246 and the insertion fork 1252 are telescopically received within the elongated conduit 1249 of the outer sheath 1247 so that the insertion fork 1252 may be selectively moved between the retracted position shown in FIG. 19B and the extended position shown in FIG. 19C. In one embodiment, the fork 1252 is advanced into the extended position shown in FIG. 12C for loading a surgical fastener onto the fork. The fork is then retracted to the position shown in FIG. 19B, and the distal end of the outer sheath is advanced through tissue with the insertion fork 1252 remaining retracted within the distal end opening 1251 of the outer sheath 1247. Referring to FIG. 19C, when the distal end of the outer sheath 1247 has reached a desired location relative to an implantation site for a surgical fastener, the elongated shaft 1246 and the insertion fork 1252 are extended beyond the distal end opening 1251 at the distal end of the outer sheath 1247 for inserting a surgical fastener loaded on the fork 1252 into tissue. In one embodiment, the insertion tool 1240 having the outer sheath 1247 may be used for inserting surgical fasteners into tissue during both open and laparoscopic procedures. The outer sheath may be made of biocompatible materials such as metal, plastic, glass, etc.

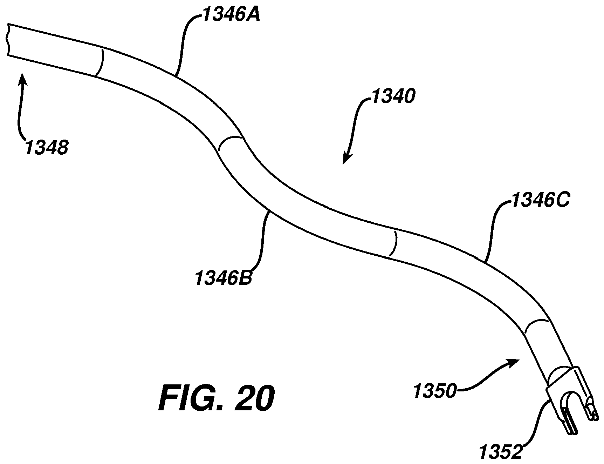

Referring to FIG. 20, in one embodiment, a surgical fastener insertion tool 1340 preferably includes an elongated shaft 1346 having a proximal end 1348 and a distal end 1350 with an insertion fork 1352 secured to the distal end 1350. In one embodiment, the insertion fork 1352 is permanently secured to the distal end 1350 of the elongated shaft 1346. In another embodiment, however, the insertion fork 1352 is removably secured to the distal end 1350 of the elongated shaft 1346.

In one embodiment, the elongated shaft 1346 desirably extends along a zig-zag or multiple curved path to enable the fork 1352 to reach specific anatomical areas of the body. In the particular embodiment shown in FIG. 20, the elongated shaft 1346 has three curved sections 1346A, 1346B, 1346C that change the direction of the elongated shaft to provide the elongated shaft with a circuitous path between the proximal end 1348 and distal end 1350 thereof.

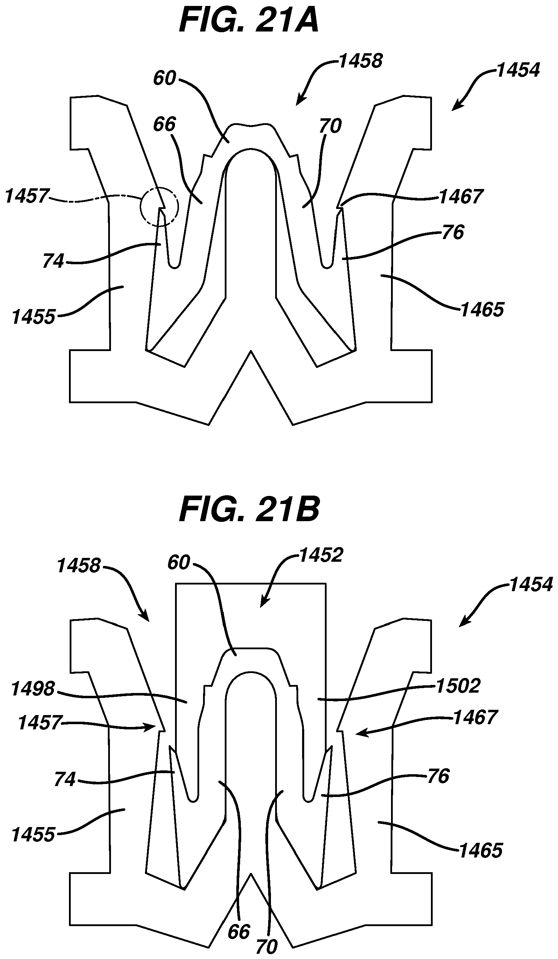

Using cartridges for holding a plurality of surgical fasteners was disclosed above in FIGS. 1A, 1B, and 1B-1. Referring to FIGS. 21A and 21B, in one embodiment, a cartridge 1454 for holding a plurality of surgical fasteners preferably has a retention feature for holding each surgical fastener 60 within the cartridge until surgical personnel desire to remove the surgical fastener 60 from the cartridge 1454. The surgical fasteners are preferably removed one at a time from the cartridge 1454.

In one embodiment, the cartridge 1454 desirably includes a first sidewall 1455, a second sidewall 1465 and a center wall 1475 that extends between the first and second sidewalls 1455, 1465. An elongated slot 1458 extends between the upper ends of the first and second sidewalls 1455, 1465 to provide access to each surgical fastener 60. In one embodiment, the cartridge 1454 preferably has a plurality of elongated slots 1458 extending along the length of the cartridge, with each elongated slot being associated with one of the plurality of surgical fasteners loaded into the cartridge.

In one embodiment, the inner face of the first sidewall 1455 desirably includes a ledge 1457 that is adapted to engage the first barb 74 of the surgical fastener 60. The inner face of the second sidewall 1454 desirably includes a second ledge 1467 that is adapted to engage the second barb 76 of the surgical fastener 60. The first and second ledges 1457, 1467 preferably hold the surgical fastener 60 in place within the cartridge until the surgical fastener is to be removed from the cartridge.

As shown in FIG. 21A, when the surgical fastener 60 is pre-loaded into the elongated slot 1458 of the cartridge 1454, the bridge 72 of the surgical fastener 60 preferably rests atop the upper end of the central wall 1475, and the proximal ends of the barbs 74, 76 are seated in the respective ledges 1457, 1467 of the first and second sidewalls 1455, 1465.

Referring to FIG. 21B, in order to remove the surgical fastener 60 from the cartridge 1454, an insertion fork 1452 is inserted into the elongated slot 1458 of the cartridge 1454. Upon insertion, the first and second tines 1498, 1502 at the distal end of the insertion fork 1452 force the first and second legs 66, 70 of the surgical fastener 60 from the first position shown in FIG. 21A (i.e., legs 66, 70 extending away from one another), to the second position shown in FIG. 21B (i.e., legs 66, 70 generally parallel with one another), whereupon the surgical fastener 60 is loaded onto the fork 1452. As a result, the proximal ends of the barbs 74, 76 are no longer constrained by the first and second ledges 1457, 1467, which enables the surgical fastener 60 to be removed from the cartridge 1454.

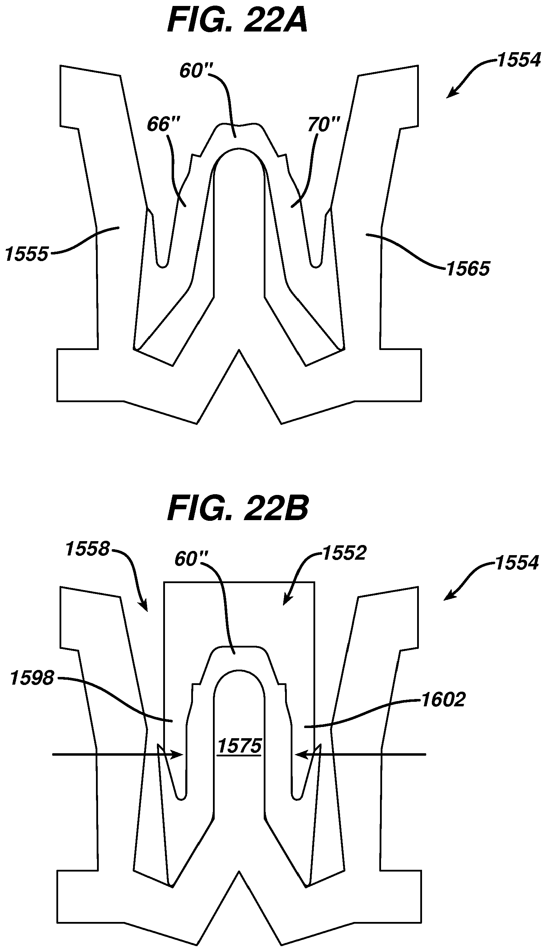

Referring to FIGS. 22A and 22B, in one embodiment, the first and second legs 66", 70" of a surgical fastener 60" are molded or annealed so that the legs normally extend away from one another at an angle, which maintains tension against the opposing inner faces of a first sidewall 1555 and a second sidewall 1565 of a cartridge 1554. When an insertion fork 1552 is inserted into the elongated slot 1558 of the cartridge 1554, the tines 1598, 1602 flex the first and second legs 66", 70" inwardly toward the central wall 1575 so that the surgical fastener 60" may be removed from the cartridge 1554. In one embodiment, the cartridge 1554 preferably contains a plurality of surgical fasteners that may be removed one at a time from the cartridge.

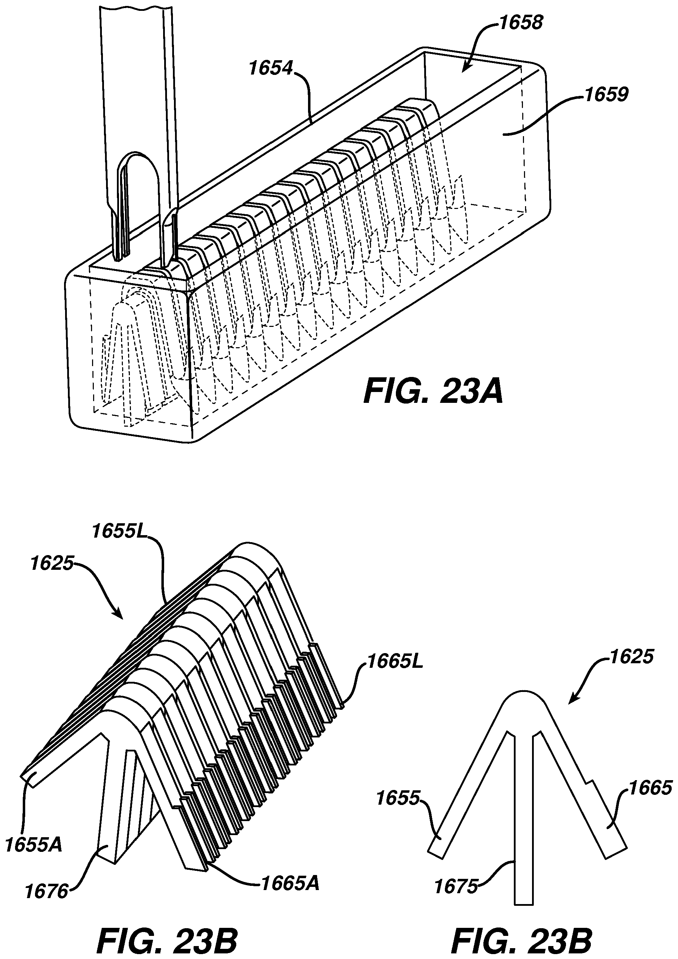

Referring to FIGS. 23A-23D, in one embodiment, a cartridge 1654 designed to hold a plurality of surgical fasteners preferably includes a top opening 1658 that provides access to a central chamber 1559 surrounded by two lateral walls and two end walls. The cartridge 1654 preferably includes a flexible member 1625 having a plurality of first flexible arms 1655A-1655L that move independently of one another, a plurality of opposing second flexible arms 1665A-1665L that move independently of one another and that are linked with one of the first flexible arms 1655A-1655L, and a central support 1675 that extends along the length of the cartridge 1654 between the first and second flexible arms.

As shown in FIG. 23A, a surgical fastener 60 is preferably positioned atop each grouping of one of the first flexible arms 1655 and one of the second flexible arms 1665 associated therewith. FIGS. 23A-23C show the normal position of the first and second flexible arms. When an insertion fork is used for removing a surgical fastener from the cartridge, the first and second flexible arms are designed to flex inwardly toward the central support 1675.

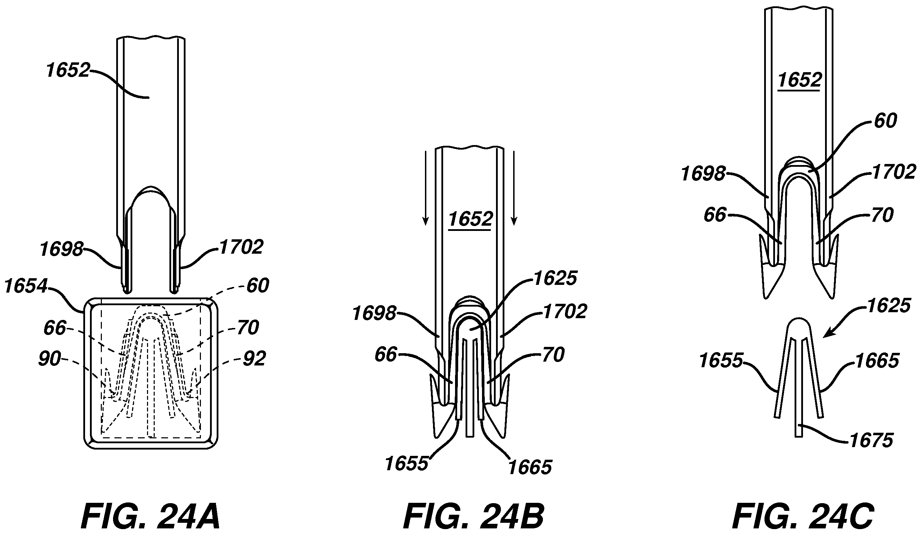

Referring to FIGS. 24A-24C, in one embodiment, a single surgical fastener 60 is removed from the cartridge 1654 (FIGS. 23A-23C) by aligning the tines 1698, 1702 at the distal end of an insertion fork 1652 with the legs 66, 70 of the surgical fastener 60. The tines 1698, 1707 are then advanced over the legs until the distal ends of the tines 1698, 1702 engage the insertion tool seating surfaces 90, 92 at the proximal ends of the insertion tips 68, 72.

Referring to FIG. 24B, as the tines 1698, 1702 slide over the first and second arms 66, 70, the tines force the first and second arms 66, 70 inwardly, which, in turn, forces the first and second flexible arms 1655, 1665 of the flexible member 1625 inwardly, whereupon the surgical fastener 60 is released from the flexible member 1625. In one embodiment, in the stage of the surgical fastener loading process shown in FIG. 24B, the first and second arms 66, 70 of the surgical fastener 60 are parallel with one another, and the first and second flexible arms 1655, 1665 of the flexible member 1625 are parallel with one another.

Referring to FIG. 24C, in one embodiment, after the surgical fastener 60 is loaded onto the tines 1698, 1702 of the insertion fork 1652, the fork is retracted from the cartridge 1654 (FIG. 24A). As the legs 66, 70 of the surgical fastener are lifted from the first and second flexible arms 1655, 1665 of the flexible member 1625, the flexible arms spring away from one another for returning to the normal position from the compressed position shown in FIG. 24B to the uncompressed position shown in FIG. 24C.

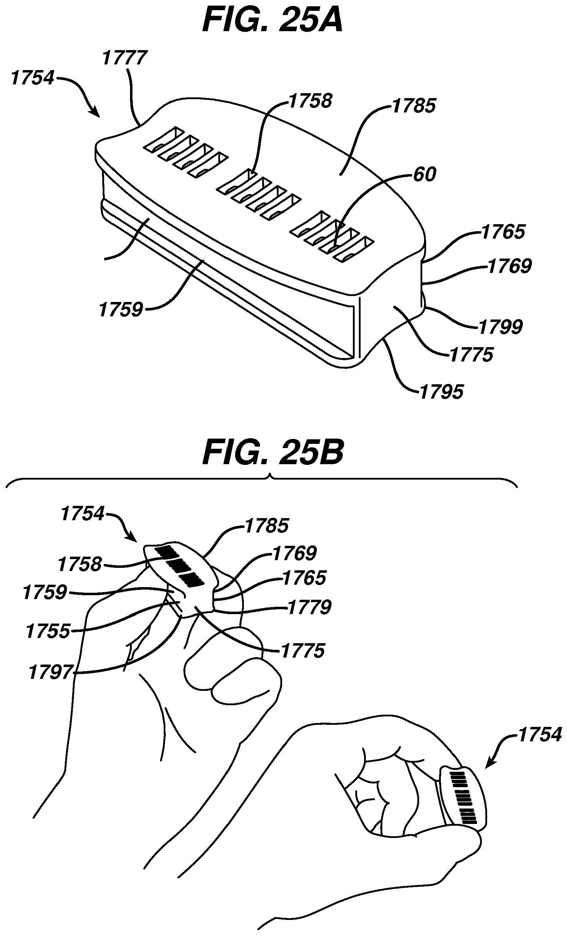

Referring to FIGS. 25A and 25B, in one embodiment, a cartridge 1754 includes a body having first and second lateral sidewalls 1755, 1765 that oppose one another and that extend along the length of the cartridge, and first and second end walls 1775, 1777 that oppose one another and that extend between the first and second lateral side walls. In one embodiment, the outer surfaces of one or more of the lateral side walls 1755, 1765, and the outer surface of one or more of the end walls 1775, 1777 may have concave surfaces that facilitate gripping and control of the cartridge by surgical personnel.

In one embodiment, the cartridge 1754 preferably includes a top plate 1785 having a plurality of elongated slots 1758 formed therein. Each of the elongated slots 1758 preferably provides access to a single surgical fastener 60 (FIG. 2A, FIG. 5) that is pre-loaded within the cartridge 1754. In one embodiment, the cartridge 1754 desirably has a bottom plate 1795 that extends along the bottom of the cartridge. The top plate 1785 and the bottom plate 1795 preferably have lateral edges that extend laterally beyond the respective first and second sidewalls 1755, 1765 of the cartridge to provide protective flanges and/or mounting rails that extend along the respective top and bottom of the cartridge. In one embodiment, the laterally extending edges on the bottom plate 1795 preferably define first and second rails 1797, 1799 that enable the cartridge 1754 to interface with securing elements on a base plate so that the cartridge may be securely mounted on the base plate, and then easily removed from the base plate, if desired. In one embodiment, when the cartridge is held by a user's fingers, the first and second laterally extending edges on the top plate 1785 protect the user's fingers as the fork of an insertion tool is inserted into one of the elongated slots 1758. The first and second laterally extending edges may also enhance control of the cartridge 1754.

In one embodiment, the cartridge 1754 preferably includes a first side channel 1759 that extends along the length of the first lateral sidewall 1755 of the cartridge, and between the first lateral edge of the top plate 1785 and the first rail 1797 of the bottom plate 1795. The cartridge also preferably includes a second side channel 1769 that extends along the length of the second lateral sidewall 1769, and between the second lateral edge of the top plate 1785 and the second bottom rail 1799 of the bottom plate 1795.

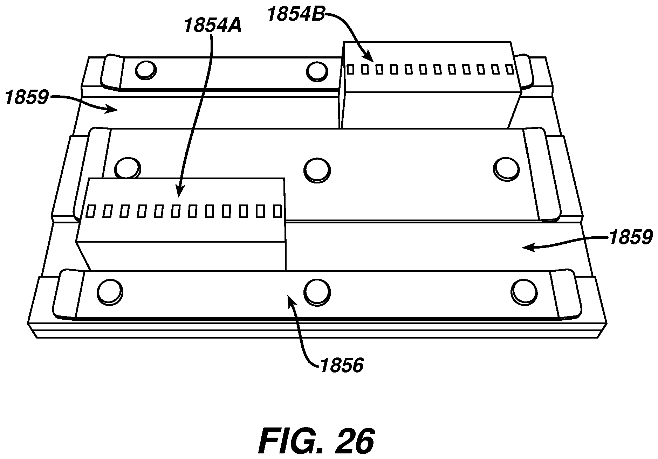

Referring to FIG. 26, in one embodiment, a support base 1856 is utilized for securing one or more of the cartridges disclosed herein to the base. In one embodiment, the base 1856 preferably includes one or more plates 1857 secured over a top face of the base 1856. Elongated slots 1859 desirably extend between the plates 1857. The width of the elongated slots preferably matches the distance between the first and second lateral side walls of a cartridge and the rails at the bottom plate of the cartridge preferably define a distance that is greater than the width of the elongated slots 1859. In one embodiment, the rails extending along the bottom of the cartridge 1854 are desirably slid into the elongated slots 1859 for securely holding the cartridges to the base 1856.

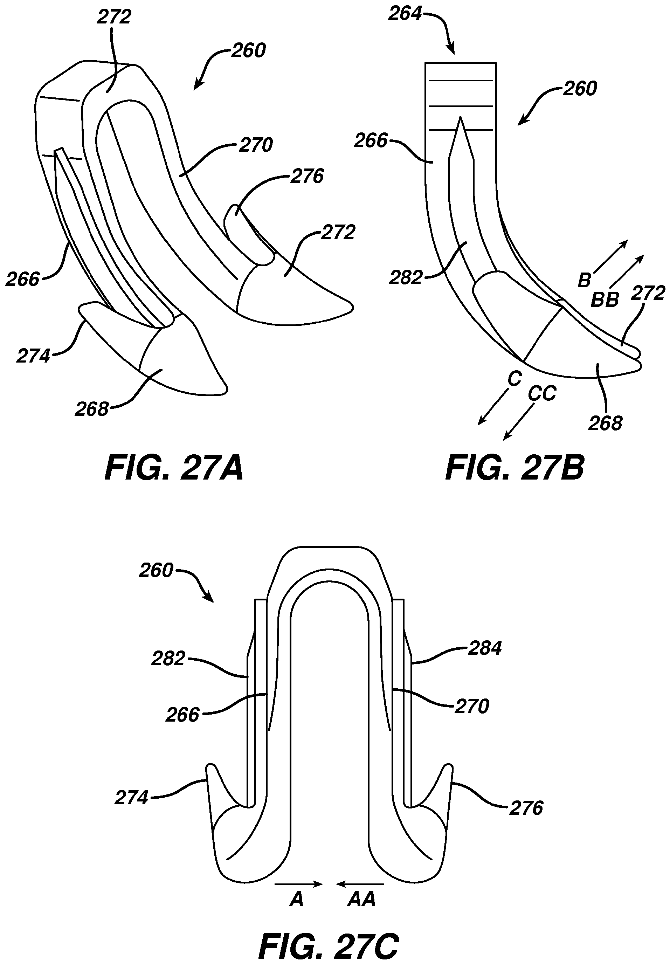

Referring to FIGS. 27A-27C, in one embodiment, a surgical fastener 260 preferably includes a first leg 266, a second leg 270 and a bridge 272 that interconnects the proximal ends of the first and second legs 266, 270. The surgical fastener 260 includes insertion tips 268, 272 provided at the distal most ends of the first and second legs 266, 270. In one embodiment, the first insertion tip 268 has a first barb 274, and the second insertion tip 272 has a second barb 276. Although only one barb is shown on each insertion tip, in other embodiments, each of the insertion tips may have two or more barbs. The first and second legs 266, 270 curve between the bridge 272 and the insertion tips 268, 272 at the distal end of the surgical fastener. The first and second legs also have respective ribs 282, 284 that extend along the lengths of the respective legs 266, 270. The ribs 282, 284 are also curved and preferably mirror the curvature of the first and second legs.

In the embodiment shown in FIG. 27C, the first and second barbs 274, 276 extend away from one another. In one embodiment, the barbs provided at the distal ends of the first and second legs 266, 260 may extend toward one another as indicated by the arrows A, AA. Referring to FIG. 27B, in one embodiment, the barbs on each of the respective first and second legs may extend in the same direction and/or up from the top surfaces of the respective legs, as indicated by the arrows B, BB ("up"). Referring to FIG. 27B, in one embodiment, the barbs on each of the respective first and second legs may extend in the same direction and/or down from the bottom surfaces of the respective legs, as indicated by the arrows C, CC ("down").

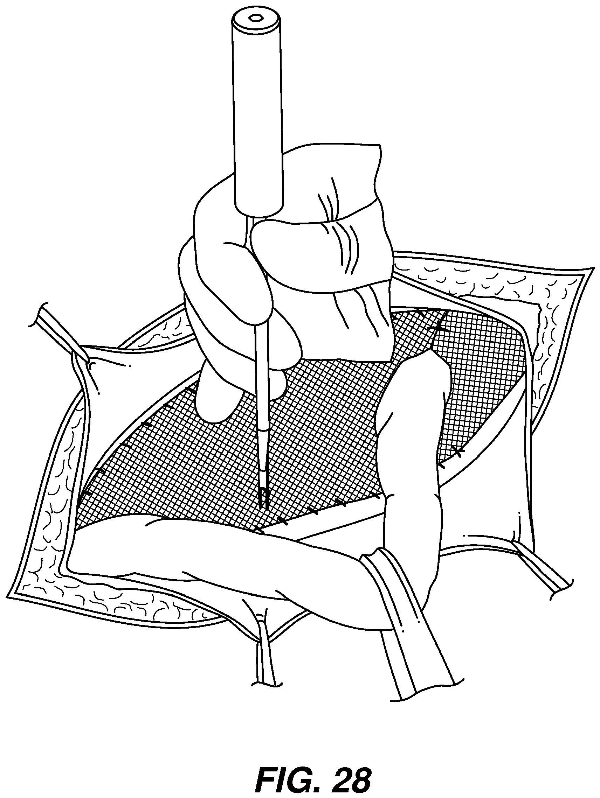

Referring to FIG. 28, in one embodiment, the surgical fastener insertion tools and the surgical fasteners disclosed herein may be utilized during an open inguinal procedure for securing an implant to tissue.

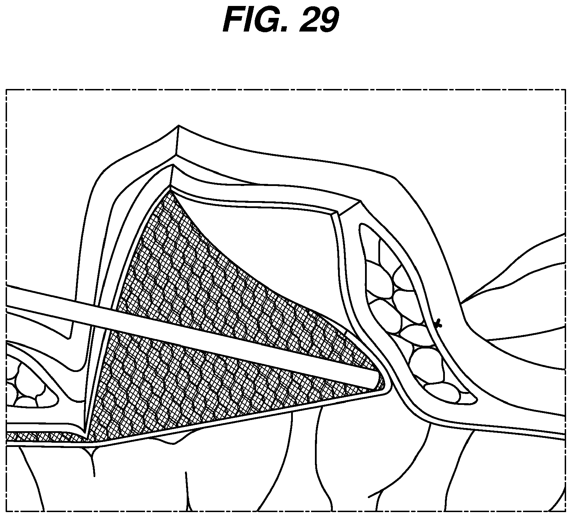

Referring to FIG. 29, in one embodiment, the surgical fastener insertion tools and the surgical fasteners disclosed herein may be utilized during an open ventral fixation procedure. In one embodiment, the surgical fastener and the insertion fork are covered by a protective outer sheath until the distal end of the sheath reaches a desired location, and the insertion fork with the surgical fastener loaded thereon is extended beyond the distal end of the outer sheath for securing a surgical implant to tissue.

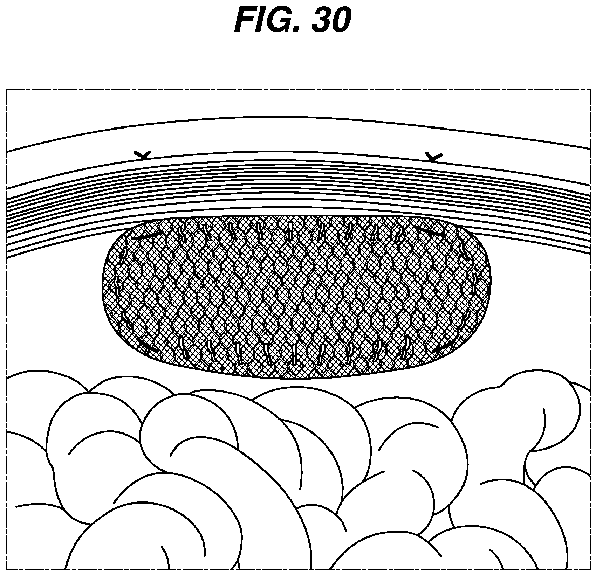

Referring to FIG. 30, in one embodiment, the surgical fastener insertion tools and the surgical fasteners disclosed herein may be utilized for a laparoscopic procedure to secure a surgical implant to tissue.

The headings used herein are for organizational purposes only and are not meant to be used to limit the scope of the description or the claims. As used throughout this application, the word "may" is used in a permissive sense (i.e., meaning having the potential to), rather than the mandatory sense (i.e., meaning must). Similarly, the words "include", "including", and "includes" mean including but not limited to. To facilitate understanding, like reference numerals have been used, where possible, to designate like elements common to the figures.

While the foregoing is directed to embodiments of the present invention, other and further embodiments of the invention may be devised without departing from the basic scope thereof, which is only limited by the scope of the claims that follow.

## Claims

1. An applicator system for surgical fasteners (60) comprising:
an insertion tool (240) having a shaft (242) with a proximal end (248), a distal end (250), and an insertion fork (252) connected to the distal end (250) of said shaft (242);
at least one cartridge (54) containing a plurality of surgical fasteners (60), wherein said surgical fasteners (60) are removable one at a time from said cartridge (54) by engaging one of said surgical fasteners (60) with said insertion fork (252) and removing said engaged surgical fastener (60) from said cartridge (54);
and a surgical instrument (165) or a handle (442),
wherein said surgical instrument (165) has a longitudinal axis (A₁) and clamping jaws (167, 169) at a distal end thereof, wherein said surgical instrument (165) is adapted to hold said insertion tool (240) between said clamping jaws (167, 169) such that the shaft (242) of the insertion tool (240) extends laterally along a second axis that traverses the longitudinal axis (A₁) of said surgical instrument (165),
wherein said handle (442) has a longitudinal axis (A₁), a proximal (448) end and a distal end (450), wherein said insertion tool (240) is permanently secured to the distal end (450) of said handle (442) such that the shaft (242) of the insertion tool (240) extends laterally along a second axis that traverses the longitudinal axis (A₁) of said handle (442).

2. The applicator system as claimed in claim 1, wherein said shaft (242) of said insertion tool (240) is curved.

3. The applicator system as claimed in claim 1, wherein said shaft (242) of said insertion tool (240) is angled or straight.

4. The applicator system as claimed in claim 1, wherein said second axis of said shaft (242) of said insertion tool (240) defines an angle with said longitudinal axis of said surgical instrument (165) or said handle (442).

5. The applicator system as claimed in claim 1, wherein said second axis of said shaft (242) of said insertion tool (240) is perpendicular to said longitudinal axis (A₁) of said surgical instrument (165) or said handle (442).

6. The applicator system as claimed in claim 4, wherein said angle between said second axis of said insertion tool (240) and said longitudinal axis (A₁) of said surgical instrument (165) or said handle (442) is an acute or an obtuse angle.

7. The applicator system as claimed in claim 1, wherein each said surgical fastener (60) comprises:
a first leg (66) having a proximal end and a distal end;
a first insertion tip (68) at the distal end of said first leg (66);
a second leg (70) having a proximal end and a distal end;
a second insertion tip (72) at the distal end of said second leg (70);
a bridge (72) interconnecting and extending between the proximal ends of said first (66) and second (70) legs.

8. The applicator system as claimed in claim 7, wherein said first (66) and second (70) legs are straight or curved.

9. The applicator system as claimed in claim 7, wherein said insertion fork (252) has first (98) and second (102) tines extending from a distal end (96) thereof, and wherein said first tine (98) engages said first leg (66) and said second tine (102) engages said second leg (70) for loading said surgical fastener (60) onto said insertion fork (252) for being removed from said cartridge (54).

10. The applicator system as claimed in claim 9, wherein said first leg (66) has a first alignment rib (82) that extends between the proximal and distal ends thereof and said second leg (70) has a second alignment rib (84) extending between the proximal and distal ends thereof.

11. The applicator system as claimed in claim 10, wherein said first tine (98) has a first concave groove (100), and said second tine (102) has a second concave groove (104), and wherein said first (100) and second (104) grooves slide over said first (82) and second (84) alignment ribs of said first (66) and second (70) legs, respectively, for loading said surgical fastener (60) onto said insertion fork (252).

12. The applicator system as claimed in claim 11, wherein said first (82) and second (84) alignment ribs have a first height (H₁) and said first (100) and second (104) concave grooves have a second height (H₂) that is greater than the first height (H₁) of said alignment ribs (82, 84).

13. The applicator system as claimed in claim 11, wherein each said alignment rib (82, 84) has a wider distal section (84B') defining a third height (H₃) that is greater than the second height (H₂) of said first (100) and second (104) concave grooves on said tines (98, 102) for forming an interference fit between said tines (98, 102) and said wider distal sections (84B') of said alignment ribs (82, 84).

14. The applicator system as claimed in claim 1, wherein said at least one cartridge (54) has a plurality of slots (1758) formed therein, and wherein one of said surgical fasteners (60) is contained in each of said slots (1758).

15. The applicator system as claimed 1, wherein said at least one cartridge (54) comprises two or more cartridges (1854A, 1854B) containing surgical fasteners (60), and wherein the properties of said surgical fasteners (60) in each said cartridge (1854A, 1854B) are different, optionally wherein said surgical fasteners (60) contained within a first one (1854A) of said cartridges (1854A, 1854B) are larger than said surgical fasteners (60) contained within a second one (1854B) of said cartridges.

## Patentansprüche

1. Applikatorsystem für chirurgische Befestigungsvorrichtungen (60), umfassend:
ein Einführwerkzeug (240), das einen Schaft (242) mit einem proximalen Ende (248), einem distalen Ende (250) und einer mit dem distalen Ende (250) des Schafts (242) verbundenen Einführgabel (252) hat,
mindestens ein Magazin (54), das eine Vielzahl von chirurgischen Befestigungsvorrichtungen (60) enthält, wobei die chirurgischen Befestigungsvorrichtungen (60) nacheinander aus dem Magazin (54) entfernbar sind, indem eine der chirurgischen Befestigungsvorrichtungen (60) mit der Einführgabel (252) in Eingriff genommen wird und die in Eingriff genommene chirurgische Befestigungsvorrichtung (60) aus dem Magazin (54) entfernt wird,
und ein chirurgisches Instrument (165) oder einen Griff (442),
wobei das chirurgische Instrument (165) eine Längsachse (A₁) und Klemmbacken (167, 169) an einem distalen Ende davon hat, wobei das chirurgische Instrument (165) geeignet ist, das Einführwerkzeug (240) zwischen den Klemmbacken (167, 169) zu halten, so dass sich der Schaft (242) des Einführwerkzeugs (240) seitlich entlang einer zweiten Achse erstreckt, die die Längsachse (A₁) des chirurgischen Instruments (165) überquert,
wobei der Griff (442) eine Längsachse (A₁), ein proximales Ende (448) und ein distales Ende (450) hat, wobei das Einführwerkzeug (240) permanent am distalen Ende (450) des Griffs (442) befestigt ist, so dass sich der Schaft (242) des Einführwerkzeugs (240) seitlich entlang einer zweiten Achse erstreckt, die die Längsachse (A₁) des Griffs (442) überquert.

2. Applikatorsystem nach Anspruch 1, wobei der Schaft (242) des Einführwerkzeugs (240) gekrümmt ist.

3. Applikatorsystem nach Anspruch 1, wobei der Schaft (242) des Einführwerkzeugs (240) abgewinkelt oder gerade ist.

4. Applikatorsystem nach Anspruch 1, wobei die zweite Achse des Schafts (242) des Einführwerkzeugs (240) einen Winkel mit der Längsachse des chirurgischen Instruments (165) oder des Griffs (442) definiert.

5. Applikatorsystem nach Anspruch 1, wobei die zweite Achse des Schafts (242) des Einführwerkzeugs (240) senkrecht zu der Längsachse (A₁) des chirurgischen Instruments (165) oder des Griffs (442) verläuft.

6. Applikatorsystem nach Anspruch 4, wobei der Winkel zwischen der zweiten Achse des Einführwerkzeugs (240) und der Längsachse (A₁) des chirurgischen Instruments (165) oder des Griffs (442) ein spitzer oder ein stumpfer Winkel ist.

7. Applikatorsystem nach Anspruch 1, wobei jede chirurgische Befestigungsvorrichtung (60) Folgendes umfasst:
einen ersten Schenkel (66) mit einem proximalen Ende und einem distalen Ende,
eine erste Einführspitze (68) am distalen Ende des ersten Schenkels (66),
einen zweiten Schenkel (70) mit einem proximalen Ende und einem distalen Ende,
eine zweite Einführspitze (72) am distalen Ende des zweiten Schenkels (70),
eine Brücke (72), die die proximalen Enden des ersten (66) und des zweiten (70) Schenkels verbindet und sich dazwischen erstreckt.

8. Applikatorsystem nach Anspruch 7, wobei der erste (66) und der zweite (70) Schenkel gerade oder gekrümmt sind.

9. Applikatorsystem nach Anspruch 7, wobei die Einführgabel (252) eine erste (98) und eine zweite (102) Zinke hat, die sich von einem distalen Ende (96) davon erstrecken, und wobei die erste Zinke (98) den ersten Schenkel (66) in Eingriff nimmt und die zweite Zinke (102) den zweiten Schenkel (70) in Eingriff nimmt, um die chirurgische Befestigungsvorrichtung (60) zur Entfernung aus dem Magazin (54) auf die Einführgabel (252) zu laden.

10. Applikatorsystem nach Anspruch 9, wobei der erste Schenkel (66) eine erste Ausrichtungsrippe (82) hat, die sich zwischen dem proximalen und dem distalen Ende davon erstreckt, und der zweite Schenkel (70) eine zweite Ausrichtungsrippe (84) hat, die sich zwischen dem proximalen und dem distalen Ende davon erstreckt.

11. Applikatorsystem nach Anspruch 10, wobei die erste Zinke (98) eine erste konkave Nut (100) hat und die zweite Zinke (102) eine zweite konkave Nut (104) hat, und wobei die erste (100) und die zweite (104) Nut über die erste (82) bzw. die zweite (84) Ausrichtungsrippe des ersten (66) und des zweiten (70) Schenkels gleiten, um die chirurgische Befestigungsvorrichtung (60) auf die Einführgabel (252) zu laden.

12. Applikatorsystem nach Anspruch 11, wobei die erste (82) und die zweite (84) Ausrichtungsrippe eine erste Höhe (H₁) haben und die erste (100) und die zweite (104) konkave Nut eine zweite Höhe (H₂) haben, die größer als die erste Höhe (H₁) der Ausrichtungsrippen (82, 84) ist.

13. Applikatorsystem nach Anspruch 11, wobei jede Ausrichtungsrippe (82, 84) einen breiteren distalen Abschnitt (84B') hat, der eine dritte Höhe (H₃) definiert, die größer als die zweite Höhe (H₂) der ersten (100) und der zweiten (104) konkaven Nut an den Zinken (98, 102) ist, um eine Presspassung zwischen den Zinken (98, 102) und den breiteren distalen Abschnitten (84B') der Ausrichtungsrippen (82, 84) zu bilden.

14. Applikatorsystem nach Anspruch 1, wobei das mindestens eine Magazin (54) eine Vielzahl von darin ausgebildeten Schlitzen (1758) hat und wobei eine der chirurgischen Befestigungsvorrichtungen (60) in jedem der Schlitze (1758) enthalten ist.

15. Applikatorsystem nach Anspruch 1, wobei das mindestens eine Magazin (54) zwei oder mehr Magazine (1854A, 1854B), die chirurgische Befestigungsvorrichtungen (60) enthalten, und wobei die Eigenschaften der chirurgischen Befestigungsvorrichtungen (60) in jedem Magazin (1854A, 1854B) unterschiedlich sind, wahlweise wobei die in einem ersten (1854A) der Magazine (1854A, 1854B) enthaltenen chirurgischen Befestigungsvorrichtungen (60) größer als die in einem zweiten (1854B) der Magazine enthaltenen chirurgischen Befestigungsvorrichtungen (60) sind.

## Revendications

1. Système d'applicateur pour pinces chirurgicales (60) comprenant :
un outil d'insertion (240) ayant une tige (242) avec une extrémité proximale (248), une extrémité distale (250) et une fourche d'insertion (252) reliée à l'extrémité distale (250) de ladite tige (242) ;
au moins une cartouche (54) contenant une pluralité de pinces chirurgicales (60), dans laquelle lesdites pinces chirurgicales (60) peuvent être retirées les unes après les autres de ladite cartouche (54) en prenant une desdites pinces chirurgicales (60) avec ladite fourche d'insertion (252) et en retirant ladite pince chirurgicale (60) en prise hors de ladite cartouche (54) ; et
un instrument chirurgical (165) ou une poignée (442) ;
dans lequel ledit instrument chirurgical (165) a un axe longitudinal (A₁) et des mâchoires de serrage (167, 169) à une extrémité distale correspondante, dans lequel ledit instrument chirurgical (165) est conçu pour retenir ledit outil d'insertion (240) entre lesdites mâchoires de serrage (167, 169) de telle sorte que la tige (242) de l'outil d'insertion (240) s'étende en côté le long d'un second axe qui traverse l'axe longitudinal (A₁) dudit instrument chirurgical (165) ;
dans lequel ladite poignée (442) a un axe longitudinal (A₁), une extrémité proximale (448) et une extrémité distale (450), dans lequel ledit outil d'insertion (240) est en permanence fixé de façon sécurisée à l'extrémité distale (450) de ladite poignée (442) de telle sorte que la tige (242) de l'outil d'insertion (240) s'étende en côté le long d'un second axe qui traverse l'axe longitudinal (A₁) de ladite poignée (442).

2. Système d'applicateur selon la revendication 1, dans lequel ladite tige (242) dudit outil d'insertion (240) est incurvée.

3. Système d'applicateur selon la revendication 1, dans lequel ladite tige (242) dudit outil d'insertion (240) est en angle ou droite.

4. Système d'applicateur selon la revendication 1, dans lequel ledit second axe de ladite tige (242) dudit outil d'insertion (240) définit un angle avec ledit axe longitudinal dudit instrument chirurgical (165) ou de ladite poignée (442).

5. Système d'applicateur selon la revendication 1, dans lequel ledit second axe de ladite tige (242) dudit outil d'insertion (240) est perpendiculaire audit axe longitudinal (A₁) dudit instrument chirurgical (165) ou de ladite poignée (442).

6. Système d'applicateur selon la revendication 4, dans lequel ledit angle entre ledit second axe dudit outil d'insertion (240) et ledit axe longitudinal (A₁) dudit instrument chirurgical (165) ou ladite poignée (442) est un angle aigu ou obtus.

7. Système d'applicateur selon la revendication 1, dans lequel chaque pince chirurgicale (60) comprend :
une première jambe (66) ayant une extrémité proximale et une extrémité distale ;
un premier embout d'insertion (68) à l'extrémité distale de ladite première jambe (66) ;
une seconde jambe (70) ayant une extrémité proximale et une extrémité distale ;
un second embout d'insertion (72) à l'extrémité distale de ladite seconde jambe (70) ;
un pont (72) reliant en eux les extrémités proximales desdites première (66) et seconde (70) jambes et s'étendant entre elles.

8. Système d'applicateur selon la revendication 7, dans lequel lesdites première (66) et seconde (70) jambes sont droites ou incurvées.

9. Système d'applicateur selon la revendication 7, dans lequel ladite fourche d'insertion (252) a des première (98) et seconde (102) dents s'étendant hors d'une extrémité distale (96) correspondante et dans lequel ladite première dent (98) met en prise ladite première jambe (66) et ladite seconde dent (102) met en prise ladite seconde jambe (70) pour charger ladite pince chirurgicale (60) sur ladite fourche d'insertion (252) pour être retirée de ladite cartouche (54).

10. Système d'applicateur selon la revendication 9, dans lequel ladite première jambe (66) a une première nervure d'alignement (82) qui s'étend entre les extrémités proximale et distale correspondantes et ladite seconde jambe (70) a une seconde nervure d'alignement (84) s'étendant entre les extrémités proximale et distale correspondantes.

11. Système d'applicateur selon la revendication 10, dans lequel ladite première dent (98) a une première gorge concave (100), dans lequel ladite seconde dent (102) a une seconde gorge concave (104) et dans lequel lesdites première (100) et seconde (104) gorges glissent au-dessus desdites première (82) et seconde (84) nervures d'alignement desdites première (66) et seconde (70) jambes, respectivement, pour charger ladite pince chirurgicale (60) sur ladite fourche d'insertion (252).

12. Système d'applicateur selon la revendication 11, dans lequel lesdites première (82) et seconde (84) nervures d'alignement ont une première hauteur (H₁) et lesdites première (100) et seconde (104) gorges concaves ont une seconde hauteur (H₂) supérieure à la première hauteur (H₁) desdites nervures d'alignement (82, 84).

13. Système d'applicateur selon la revendication 11, dans lequel chaque nervure d'alignement (82, 84) a une section distale (84B') plus large définissant une troisième hauteur (H₃) supérieure à la seconde hauteur (H₂) desdites première (100) et seconde (104) gorges concaves sur lesdites dents (98, 102) pour former un ajustement d'interférence entre lesdites dents (98, 102) et lesdites sections distales (84B') plus larges desdites nervures d'alignement (82, 84).

14. Système d'applicateur selon la revendication 1, dans lequel ladite au moins une cartouche (54) a une pluralité de fentes (1758) formées à l'intérieur et dans lequel une desdites pinces chirurgicales (60) est contenue dans chacune desdites fentes (1758).

15. Système d'applicateur selon la revendication 1, dans lequel ladite au moins une cartouche (54) comprend deux cartouches (1854A, 1854B) ou plus contenant des pinces chirurgicales (60) et dans lequel les propriétés desdites pinces chirurgicales (60) dans chaque cartouche (1854A, 1854B) sont différentes, en option dans lequel lesdites pinces chirurgicales (60) contenues à l'intérieur d'une première cartouche (1854A) parmi les cartouches (1854A, 1854B) sont plus grandes que lesdites pinces chirurgicales (60) contenues à l'intérieur d'une seconde cartouche (1854B) parmi lesdites cartouches.
